# EUROPEAN PATENT SPECIFICATION

(11) **EP 2 135 618 B1**
(45) Date of publication and mention of the grant of the patent: **15.01.2014**
(21) Application number: 08016231.6
(22) Date of filing: 19.11.2004
(51) Int. Cl.: A61K 38/17, C12N 5/10, A01K 67/027, G01N 33/50, C12Q 1/68, A61P 3/00

(54) **SF6 for use in the treatment of diabetes and / or obesity, methods of screening for modulators and kit**
SF6 zur Verwendung zur Behandlung von Diabetes und/oder Fettleibigkeit, Screeningverfahren für Modulatoren und Kit
SF6 pour son utilisation dans le traitement du diabète et / ou de l'obésité, méthodes de criblage pour modulateurs et Kit

(30) Priority: 19.11.2003 EP 03026614
(43) Date of publication of application: 23.12.2009
(62) Divisional of application: 04803195.9
(73) Proprietor: Evotec International GmbH, 22419 Hamburg (DE)
(72) Inventor: Onichtchouk, Daria, 79100 Freiburg (DE)
(74) Representative: Weiss, Wolfgang

(56) References cited:
- EP-A- 1 149 844
- WO-A-2004/072100
- WO-A-2004/087194
- US-A- 5 279 966
- US-A1- 2002 107 383
- US-A1- 2002 144 296
- DATABASE GENBANK [Online] 3 December 2009 (2009-12-03), XP002558613 retrieved from GENBANK accession no. NP_058023
- DATABASE GENBANK [Online] 3 December 2009 (2009-12-03), XP002558614 retrieved from GENBANK Database accession no. NP_006150
- LUCE M J ET AL: "The neuronal EGF-related genes NELL1 and NELL2 are expressed in hemopoietic cells and developmentally regulated in the B lineage" GENE, ELSEVIER, AMSTERDAM, NL, vol. 231, no. 1-2, 29 April 1999 (1999-04-29), pages 121-126, XP004166785 ISSN: 0378-1119
- WATANABE T K ET AL: "CLONING AND CHARACTERIZATION OF TWO NOVEL HUMAN CDNAS (NELL1 AND NELL2) ENCODING PROTEINS WITH SIX EGF-LIKE REPEATS" GENOMICS, ACADEMIC PRESS, SAN DIEGO, US, vol. 38, no. 3, 15 December 1996 (1996-12-15), pages 273-276, XP002064052 ISSN: 0888-7543
- KURODA ET AL: "Biochemical Characterization and Expression Analysis of Neural Thrombospondin-1-like Proteins NELL1 and NELL2" BIOCHEMICAL AND BIOPHYSICAL RESEARCH COMMUNICATIONS, ACADEMIC PRESS INC. ORLANDO, FL, US, vol. 265, no. 1, 11 November 1999 (1999-11-11), pages 79-86, XP005100839 ISSN: 0006-291X
- KURODA S ET AL: "Involvement of epidermal growth factor-like domain of NELL proteins in the novel protein-protein interaction with protein kinase C" BIOCHEMICAL AND BIOPHYSICAL RESEARCH COMMUNICATIONS, ACADEMIC PRESS INC. ORLANDO, FL, US, vol. 265, no. 3, 1 January 1999 (1999-01-01), pages 752-757, XP003007039 ISSN: 0006-291X
- HA C ET AL: "NELL2, a novel neural tissue-specific epidermal growth factor-like repeat domain-containing protein, is involved in the preproenkephalin gene expression." SOCIETY FOR NEUROSCIENCE ABSTRACT VIEWER AND ITINERARY PLANNER, vol. 2003, 2003, pages Abstract No. 396.10 URL-http://sf, XP009126721 & 33RD ANNUAL MEETING OF THE SOCIETY OF NEUROSCIENCE; NEW ORLEANS, LA, USA; NOVEMBER 08-12, 2003
- DATABASE BIOSIS [Online] BIOSCIENCES INFORMATION SERVICE, PHILADELPHIA, PA, US; May 2001 (2001-05), DILELLA ANTHONY G ET AL: "Identification of genes differentially expressed in benign prostatic hyperplasia" XP009126707 Database accession no. PREV200100295612 & JOURNAL OF HISTOCHEMISTRY AND CYTOCHEMISTRY, vol. 49, no. 5, May 2001 (2001-05), pages 669-670, ISSN: 0022-1554
- AIHARA KOUTOKU ET AL: "A neuron-specific EGF family protein, NELL2, promotes survival of neurons through mitogen-activated protein kinases." MOLECULAR BRAIN RESEARCH, vol. 116, no. 1-2, 19 August 2003 (2003-08-19), pages 86-93, XP009126705 ISSN: 0169-328X
- NELSON B R ET AL: "Restricted neural epidermal growth factor-like like 2 (NELL2) expression during muscle and neuronal differentiation" MECHANISMS OF DEVELOPMENT 200212 IE, vol. 119, no. SUPPL. 1, December 2002 (2002-12), pages S11-S19, XP009126704 ISSN: 0925-4773
- OSHIMA KENJI ET AL: "Secretion of a peripheral membrane protein, MFG-E8, as a complex with membrane vesicles. A POSSIBLE ROLE IN MEMBRANE SECRETION" EUROPEAN JOURNAL OF BIOCHEMISTRY, BERLIN, DE, vol. 269, no. 4, February 2002 (2002-02), pages 1209-1218, XP002233407 ISSN: 0014-2956

## Description

Described herein is the use of secreted SF6 proteins, to the use of polynucleotides encoding these, and to the use of effectors/mediators thereof in the diagnosis, study, prevention, and treatment of pancreatic diseases (e.g. diabetes mellitus), obesity and/or metabolic syndrome and to the use in regeneration of tissues such as pancreatic tissues and others.

Many human proteins serve as pharmaceutically active compounds. Several classes of human proteins that serve as such active compounds include hormones, cytokines, cell growth factors, and cell differentiation factors. Most proteins that can be used as a pharmaceutically active compound fall within the family of secreted proteins. Secreted proteins are generally produced within cells at rough endoplasmic reticulum, are then exported to the golgi complex, and then move to secretory vesicles or granules, where they are secreted to the exterior of the cell via exocytosis. Examples for commercially used secreted proteins are human insulin, thrombolytic agents, interferons, interleukins, colony stimulating factors, human growth hormone, transforming growth factor beta, tissue plasminogen activator, erythropoietin, and various other proteins. Receptors of secreted proteins, which are membrane-bound proteins, also have potential as therapeutic or diagnostic agents. It is, therefore, important for developing new pharmaceutical compounds to identify secreted proteins that can be tested for activity in a variety of animal models. Thus, in light of the pervasive role of secreted proteins in human physiology, a need exists for identifying and characterizing novel functions for human secreted proteins and the genes that encode them. This knowledge will allow one to detect, to treat, and to prevent medical diseases, disorders, and/or conditions by using secreted proteins or the genes that encode them.

The pancreas is an essential organ possessing both an exocrine function involved in the delivery of enzymes into the digestive tract and an endocrine function by which various hormones are secreted into the blood stream. The exocrine function is assured by acinar and centroacinar cells that produce various digestive enzymes and intercalated ducts that transport these enzymes in alkaline solution to the duodenum. The functional unit of the endocrine pancreas is the islet of Langerhans. Islets are scattered throughout the exocrine portion of the pancreas and are composed of four cell types: alpha-, beta-, delta- and PP-cells, reviewed for example in Kim S.K. and Hebrok M., (2001) Genes Dev. 15: 111-127. Beta-cells produce insulin, represent the majority of the endocrine cells and form the core of the islets, while alpha-cells secrete glucagon and are located in the periphery. Delta-cells and PP-cells are less numerous and secrete somatostatin and pancreatic polypeptide, respectively.

Early pancreatic development has been well studied in different species, including chicken, zebrafish, and mice (for a detailed review, see Kim & Hebrok, 2001, supra). The pancreas develops from distinct dorsal and ventral anlagen. Pancreas development requires specification of the pancreas anlage along both anterior-posterior and dorsal-ventral axes. A number of transcription factors, which are critical for proper pancreatic development have been identified (see Kim & Hebrok, 2001, supra; Wilson M.E. et al., (2003) Mech Dev. 120: 65-80).

In postnatal/adult humans, the acinar and ductal cells retain a significant proliferative capacity that can ensure cell renewal and growth, whereas the islet cells become mostly mitotically inactive. This is in contrast to rodents where beta-cell replication is an important mechanism in the generation of new beta cells. It has been suggested, that during embryonic development, pancreatic islets of Langerhans originate from differentiating duct cells or other cells with epithelial morphology (Bonner-Weir S. and Sharma A., (2002) J Pathol. 197: 519-526; Gu G. et al., (2003) Mech Dev. 120: 35-43). In adult humans, new beta cells arise in the vicinity of ducts (Butler A.E. et al., (2003) Diabetes 52: 102-110; Bouwens L. and Pipeleers D.G., (1998) Diabetologia 41: 629-633). However, also an intra-islet location or an origin in the bone marrow has been suggested for precursor cells of adult beta cells (Zulewski H. et al., (2001) Diabetes 50: 521-533; lanus A. et al., (2003) J Clin Invest. 111: 843-850). Pancreatic islet growth is dynamic and responds to changes in insulin demand, such as during pregnancy or during the increase in body mass occuring during childhood. In adults, there is a good correlation between body mass and islet mass (Yoon K.H. et al., (2003) J Clin Endocrinol Metab. 88: 2300-2308).

Pancreatic beta-cells secrete insulin, which is stimulated by high blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus type I or LADA (latent autoimmue diabetes in adults (Pozzilli & Di Mario, 2001, Diabetes Care. 8:1460-67) beta-cells are being destroyed due to autoimmune attack. The amount of insulin produced by the remaining pancreatic islet cells is too low, resulting in elevated blood glucose levels (hyperglycemia). In diabetes type II, liver and muscle cells loose their ability to respond to normal blood insulin levels (insulin resistance). High blood glucose levels (and also high blood lipid levels) lead to an impairment of beta-cell function and to an increase in beta-cell apoptosis. It is interesting to note that the rate of beta-cell neogenesis does not appear to change in type II diabetics (Butler et al., 2003 supra), thus causing a reduction in total beta-cell mass over time. Eventually the application of exogenous insulin becomes necessary in type II diabetics.

Improving metabolic parameters such as blood sugar and blood lipid levels (e.g. through dietary changes, exercise, medication or combinations thereof) before beta cell mass has fallen below a critical threshold leads to a relatively rapid restoration of beta cell function. However, after such a treatment the pancreatic endocrine function would remain impaired due to the only slightly increased regeneration rate.

In type I diabetics, the lifespan of pancreatic islets is dramatically shortened due to autoimmune destruction. Treatments have been devised which modulate the immune system and may be able to stop or strongly reduce islet destruction (Raz I. et al., (2001) Lancet 358: 1749-1753; Chatenoud L. et al., (2003) Nat Rev Immunol. 3: 123-132). However, due to the relatively slow regeneration of human beta cells such treatments could only be fully successful at improving the diabetic condition if they are combined with an agent which can stimulate beta cell regeneration.

Thus, both for type I and type II diabetes (early and late stages) there is a need to find novel agents which stimulate beta cell regeneration.

Diabetes is a very disabling disease, because today's common anti-diabetic drugs do not control blood sugar levels well enough to completely prevent the occurrence of high and low blood sugar levels. Out of range blood sugar levels are toxic and cause long-term complications like for example renopathy, retinopathy, neuropathy, and peripheral vascular disease. There are also a host of related conditions, such as obesity, hypertension, heart disease, and hyperlipidemia, for which persons with diabetes are at substantially increased risk.

Apart from the impaired quality of life for the patients, the treatment of diabetes and its long-term complications presents an enormous financial burden to our healthcare systems with rising tendency. Thus, for the prevention or treatment of, type I and type II diabetes as well as for latent autoimmune diabetes in adults (LADA) there is a strong need in the art to identify factors that induce regeneration of pancreatic insulin producing beta-cells. These factors could restore normal function of the endocrine pancreas once its function is impaired or event could prevent the development or progression of diabetes type I, diabetes type II, or LADA.

Obesity is one of the most prevalent metabolic disorders in the world. It is still a poorly understood human disease that becomes as a major health problem more and more relevant for western society. Obesity is defined as a body weight more than 20% in excess of the ideal body weight, frequently resulting in a significant impairment of health. Obesity may be measured by body mass index, an indicator of adiposity or fatness. Further parameters for defining obesity are waist circumferences, skinfold thickness and bioimpedance. It is associated with an increased risk for cardiovascular disease, hypertension, diabetes mellitus type II, hyperlipidaemia and an increased mortality rate. Obesity is influenced by genetic, metabolic, biochemical, psychological, and behavioral factors and can be caused by different reasons such as non-insulin dependent diabetes, increase in triglycerides, increase in carbohydrate bound energy and low energy expenditure (Kopelman P.G., (2000) Nature 404: 635-643).

The concept of 'metabolic syndrome' (syndrome x, insulin-resistance syndrome, deadly quartet) was first described 1966 by Camus and reintroduced 1988 by Reaven (Camus J.P., (1966) Rev Rhum Mal Osteoartic 33: 10-14; Reaven G.M., (1988), Diabetes 37: 1595-1607). Today, metabolic syndrome is commonly defined as clustering of cardiovascular risk factors like hypertension, abdominal obesity, high blood levels of triglycerides and fasting glucose as well as low blood levels of HDL cholesterol. Insulin resistance greatly increases the risk of developing the metabolic syndrome (Reaven G., (2002) Circulation 106: 286-288). The metabolic syndrome often precedes the development of type II diabetes and cardiovascular disease (Lakka H.M. et al., (2002) JAMA 288: 2709-2716). The control of blood lipid levels and blood glucose levels is essential for the treatment of the metabolic syndrome (see, for example, Santomauro A.T. et al., (1999) Diabetes 48: 1836-1841).

The molecular factors regulating food intake and body weight balance are incompletely understood. Even if several candidate genes have been described which are supposed to influence the homeostatic system(s) that regulate body mass/weight, like leptin or the peroxisome proliferator-activated receptor-gamma co-activator, the distinct molecular mechanisms and/or molecules influencing obesity or body weight/body mass regulations are not known.

WO2004/087194 A2 relates to the use of a DG931 protein product for preventing and treating diabetes and/or obesity and/or metabolic syndrome.

Luce et al. (Gene 1999, vol. 231, pp.121-126) describe that the neuronal EGF-related genes NELL1 and NELL2 are expressed in hemopoietic cells and developmentally regulated in the B lineage.

Watanabe et al. (Genomics, 1996, vol. 38, pp.273-276) describe the cloning and characterization of two novel human cDNAs (NELL1 and NELL2) encoding proteins with six EGF-like repeats.

WO2004/072100 A2 relates to NELL peptide expression systems and bone formation activity of NELL peptide.

Kuroda et al. (Biochemical and Biophysical Research Communications, 1999, vol. 265, pp.79-86) describe biochemical characterization and expression analysis of neural thrombospondin-1-like proteins NELL1 and NELL2.

Kuroda et al. (Biochemical and Biophysical Research Communications, 1999, vol. 265, pp.752-757) relates to the involvement of epidermal growth factor-like domain of NELL proteins in the novel protein-protein-interaction with protein kinase C.

Ha et al., (Abstract No. 396, 33rd Annual Meeting of the Society of Neuroscience; New Orleans, LA: 2003) discloses that NELL2, a novel neural tissue-specific epidermal growth factor-like repeat domain containing protein, is involved in the preproenkephalin gene expression.

DiLella et al. (The Journal of Histochemistry and Cytochemistry, 2001, vol. 49, pp. 669-670) describe the identification of genes differentially expressed in benign prostetic hyperplasia.

Aihara et al. (Molecular Brain Research, 2003, vol. 116, pp.86-93) report that a neuron-specific EGF family protein, NELL2, promotes survival of neurons through mitogen-activated protein kinases.

Nelson et al. (Mechanisms of Development, 2002, vol. 119S, pp. S11-S19) relates to restricted neural epidermal growth factor-like like 2 (NELL2) expression during muscle and neuronal differentiation.

US 2002/107383 A1 relates to a gene useful as an indicator in the prophylaxis, diagnosis and treatment of diseases in humans.

In this invention, we disclose secreted factors referred to as SF-1-SF8, which are involved in pancreas development, regeneration, and in the regulation of energy homeostasis.

No functional data are available in the scientific prior art for the secreted protein referred to as SF1. However, the SF1 protein is structurally related to chitinases. The fly homologue of SF1 lost its enzymatic activity. It is expressed in the fat body of the Drosophila embryo and larva, and might act as a growth factor (Kawamura K. et al., (1999) Development 126: 211-219).

No functional data are available in the scientific prior art for the secreted protein referred to as SF2. Expressed sequence tags (ESTs) of the SF2 mRNA are present, e.g. in pancreas and ES cells. The SF2 gene is highly conserved in mouse, rat, and humans.

The extracellular matrix protein SF3 is involved in the growth and guidance of axons in the spinal cord and the PNS (Klar A. et al., (1992) Cell. 69: 95-110). The human SF3 gene is located at the region of chromosome 11 (between markers D11S926 and D11S928); deletion of this region causes nesidioblastosis (hyperinsulinemia) in humans. SF3 was shown to affect the biological activities of vascular endothelial cells by inhibiting angiogenesis (Terai Y. et al., (2001) J Cell Physiol. 188: 394-402). Furthermore SF3 was shown to function as a contact-repellent molecule for embryonic motor neurons (Tzarfati-Majar V. et al., (2001) Proc Natl Acad Sci U S A. 98: 4722-4727).

SF4 belongs to a structural family of neuropeptides that are widely distributed in the body and function as neurotransmitters and neuromodulators (Goto T. and Tanaka T., (2002) Microsc Res Tech. 58: 91-97). A regulatory role for SF4 in placental physiology and pre-eclampsia was described (Page N.M. et al., (2001) Regul Pept. 98: 97-104).

SF5 is a glycoprotein involved in cell anchorage and integrin signaling. SF5 is secreted from mammary gland and expressed in outer mesenchymal stromal region of the mouse fetal gonad (Oshima K. et al., (1999) Biochem Biophys Res Commun. 254: 522-528; Kanai Y. et al., (2000) Mech Dev. 96: 223-227). A possible role of SF5 in the membrane vesicle secretion and exocytosis of endocytic multivesicular bodies was suggested (Oshima K. et al., (2002) Eur J Biochem. 269: 1209-1218).

SF6 is a secreted glycoprotein expressed in CNS, PNS, and subsets of mesenchymal cells (Nelson B.R. et al., (2002) Gene Expr Patterns. 2: 7-15). SF6 contains a calcium ion binding domain.

SF7 is a serine protease inhibitor, that regulates plasma levels of glucocorticoids. Moreover SF7 is the main carrier of glucocorticoids in mammals. The temporal and spatial changes in the localization of SF7 and its mRNA in the fetus are suggested to influence the effects of steroid hormones on developing tissues (Scrocchi L.A. et al., (1993) Endocrinology. 132: 903-909). Furthermore it was suggested that during severe stress, SF7 might be important in regulating the amount of cortisol reaching target tissues (Garrel D.R. (1996) Horm Res. 45: 245-251).

SF8 is a cyclosporin A binding protein, which is expressed in a restricted subset of tissues including mouse ovary, testis, bone marrow, and kidney, and human kidney, pancreas, skeletal muscle, heart, lung, and liver (Friedman J. et al., (1994) Am J Pathol. 144: 1247-1256; Schneider H. et al., (1994) Biochemistry. 33: 8218-8224). SF8 is involved in degradation of the genome during apoptosis (Montague J.W. et al., (1997) J Biol Chem. 272: 6677-6684).

Accordingly, the present disclosure relates to secreted proteins with novel functions in the human metabolism, regeneration, and pancreatic developmental processes.

The present disclosure provides specific genes and proteins encoded thereby and effectors/modulators thereof involved in the regulation of pancreatic function and metabolism, especially in pancreas diseases such as diabetes mellitus, e.g. insulin dependent diabetes mellitus and/or non insulin dependent diabetes mellitus, and/or LADA and/or metabolic syndrome, obesity, and/or related disorders such as coronary heart disease, eating disorder, cachexia, hypertension, hypercholesterolemia (dyslipidemia), liver fibrosis, and/or gallstones. Further, the present disclosure provides specific genes and proteins encoded thereby and effectors/modulators thereof involved in the regeneration of pancreatic cells or tissues, e.g. cells having exocrinous functions such as acinar cells, centroacinar cells and/or ductal cells and/or cells having endocrinous functions, particularly cells in Langerhans islets such as alpha-, beta-, delta- and/or PP-cells, more particularly beta-cells.

We used a screen for secreted factors expressed in developing mammalian (mouse) pancreas, as described in more detail in the Examples section (see Example 1). This screen identified SF1-SF8 as secreted factors expressed in developing mouse pancreas. The present invention describes mammalian SF6 proteins and the polynucleotides encoding these, in particular human SF6, as being involved in the conditions and processes mentioned above.

The present invention is defined in the claims.

The present invention relates to SF6 polynucleotides encoding polypeptides with novel functions in the development and regeneration of pancreatic tissues and thus in mammalian pancreatic diseases (e.g. diabetes), and also in body-weight regulation, energy homeostasis, and obesity, fragments of said polynucleotides, polypeptides encoded by said polynucleotides or fragments thereof. The invention also relates to vectors, host cells, and recombinant methods for producing the polypeptides and polynucleotides for use according to the invention. Also provided are effectors/modulators of SF6 polynucleotides and/or polypeptides seletcted. from antibodies specific for a SF6 protein, antisense molecules, RNAi molecules or ribozymes, aptamers recognising said polynucleotides or polypeptides, for use according to the invention.

SF1-SF8 homologous proteins and nucleic acid molecules coding therefore are obtainable from vertebrate species. Particularly preferred are nucleic acids encoding the human SF6 protein and variants thereof. The invention particularly relates to a pharmaceutical composition for use in the treatment of diabetes and/or obesity, comprising an SF6 protein and/or a nucleic acid molecule encoding an SF6 protein and/or an effector/modulator of said nucleic acid molecule and/or said protein, wherein the nucleic acid molecule is a mammalian SF6 nucleic acid, particularly encoding a human SF6 polypeptide and/or a nucleic acid molecule, which is complementary thereto or a fragment thereof or constructs expressing anti-sense and/or dominant-negative mutated molecules, and wherein the composition optionally contains pharmaceutically acceptable carriers, diluents, and/or additives, wherein the effector/modulator is selected from antibodies which are specific for a SF6 protein, antisense molecules, aptamers, RNAi molecules and/or ribozymes recognising said SF6 protein or nucleic acid molecule encoding an SF6 protein.

Also disclosed are nucleic acid molecules encoding a polypeptide contributing to regulating the energy homeostasis and the mammalian metabolism, wherein the nucleic acid molecule comprises
(a) the nucleotide sequence of human SF1-SF8 and/or a sequence complementary thereto,
(b) a nucleotide sequence which hybridizes at 50°C in a solution containing 1 x SSC and 0.1 % SDS to a sequence of (a),
(c) a sequence corresponding to the sequences of (a) or (b) within the degeneration of the genetic code,
(d) a sequence which encodes a polypeptide which is at least 85%, preferably at least 90%, more preferably at least 95%, more preferably at least 98% and up to 99,6% identical to the amino acid sequences of the human SF1-SF8 proteins,
(e) a sequence which differs from the nucleic acid molecule of (a) to (d) by mutation and wherein said mutation causes an alteration, deletion, duplication and/or premature stop in the encoded polypeptide or
(f) a partial sequence of any of the nucleotide sequences of (a) to (e) having a length of 15-25 bases, preferably 25-35 bases, more preferably 35-50 bases and most preferably at least 50 bases.

The function of the mammalian SF1-SF8 in mammalian metabolism was validated by analyzing the expression of the transcripts in different tissues and by analyzing the role in adipocyte differentiation (see Examples 3 and 4 for more detail).

Expression profiling studies (see Examples for more detail) confirm the particular relevance of SF1-SF8 as regulators of energy metabolism in mammals.

We used mouse models of insulin resistance and/or diabetes, such as mice carrying gene knockouts in the leptin pathway (for example, ob/ob (leptin) or db (leptin receptor/ligand) mice) to study the expression of SF1-SF8. Such mice develop typical symptoms of diabetes, show hepatic lipid accumulation and frequently have increased plasma lipid levels (see Bruning J.C. et al., (1998) Mol. Cell. 2: 559-569). Expression of SF1-SF8 mRNA was also examined in susceptible wild type mice (for example, C57BI/6) that show symptoms of diabetes, lipid accumulation, and high plasma lipid levels, if fed a high fat diet. Further, expression of SF4 mRNA was also examined in Non-Obese-Diabetic (NOD) mice.

Microarrays are analytical tools routinely used in bioanalysis. A microarray has molecules distributed over, and stably associated with, the surface of a solid support. The term "microarray" refers to an arrangement of a plurality of polynucleotides, polypeptides, antibodies, or other chemical compounds on a substrate. Microarrays of polypeptides, polynucleotides, and/or antibodies have been developed and find use in a variety of applications, such as monitoring gene expression, drug discovery, gene sequencing, gene mapping, bacterial identification, and combinatorial chemistry. One area in particular in which microarrays find use is in gene expression analysis (see Example 4). Array technology can be used to explore the expression of a single polymorphic gene or the expression profile of a large number of related or unrelated genes. When the expression of a single gene is examined, arrays are employed to detect the expression of a specific gene or its variants. When an expression profile is examined, arrays provide a platform for identifying genes that are tissue specific, are affected by a substance being tested in a toxicology assay, are part of a signaling cascade, carry out housekeeping functions, or are specifically related to a particular genetic predisposition, condition, disease, or disorder.

Microarrays may be prepared, used, and analyzed using methods known in the art (see for example, Brennan T.M., (1995) U.S. Patent No. 5,474,796; Schena M. et al., (1996) Proc. Natl. Acad. Sci. USA 93:10614-10619; Baldeschwieler J.D. et al., (1995) PCT application WO 95/251116; Shalon T.D. and Brown P.O., (1995) PCT application WO 95/35505; Heller R.A. et al., (1997) Proc. Natl. Acad. Sci. USA 94: 2150-2155; Heller, M.J. and Tu E., (1997) U.S. Patent No. 5,605,662). Various types of microarrays are well known and thoroughly described in Schena M., ed. (1999; DNA Microarrays: A Practical Approach, Oxford University Press, London).

Oligonucleotides or longer fragments derived from any of the polynucleotides described herein may be used as elements on a microarray. The microarray can be used in transcript imaging techniques, which monitor the relative expression levels of large numbers of genes simultaneously as described below. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents, which are highly effective and display the fewest side effects may be selected for a patient based on his/her pharmacogenomic profile.

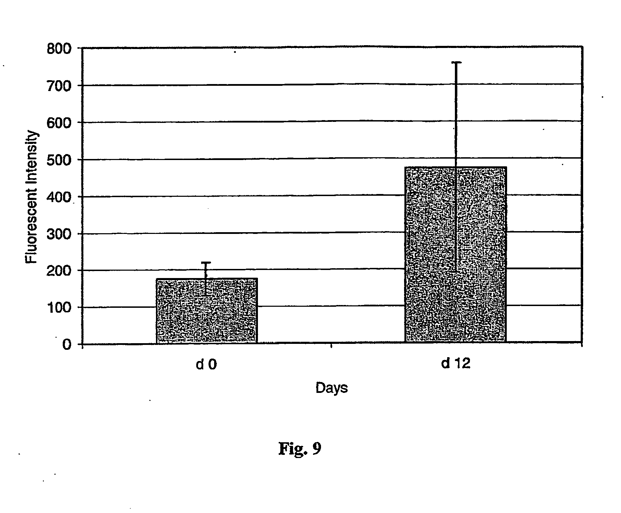

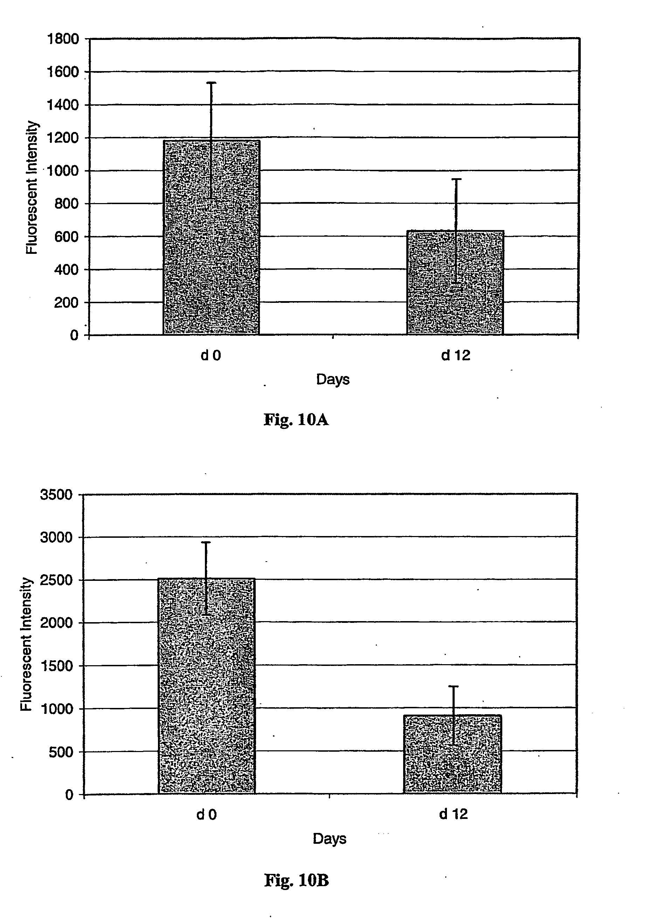

As determined by microarray analysis, SF3 and SF5 show differential expression in human adipocytes. A strong up-regulation is observed concerning the expression of SF3 during the human primary adipocyte differentiation (see Fig. 9). A strong down-regulation is observed concerning the expression of SF5 during the human primary adipocyte differentiation (see Fig. 10A) and human SGBS cell differentiation (see Fig. 10B). The SF3 protein in preadipocyctes has the potential to enhance adipocyte differentiation at a very early stage, and the SF5 protein in preadipocyctes has the potential to inhibit adipocyte differentiation at a very early stage. Therefore, the SF3 and SF5 proteins might play an essential role in adipogenesis. The results are suggesting a role of SF3 and SF5 in the regulation in human metabolism, for example, as effectors/modulators (for example, inhibitors or enhancers) of adipogenesis. Thus, SF3 and SF5 are strong candidates for the manufacture of pharmaceutical compositions and medicaments for the treatment of conditions related to human metabolism, such as diabetes, obesity, and/or metabolic syndrome.

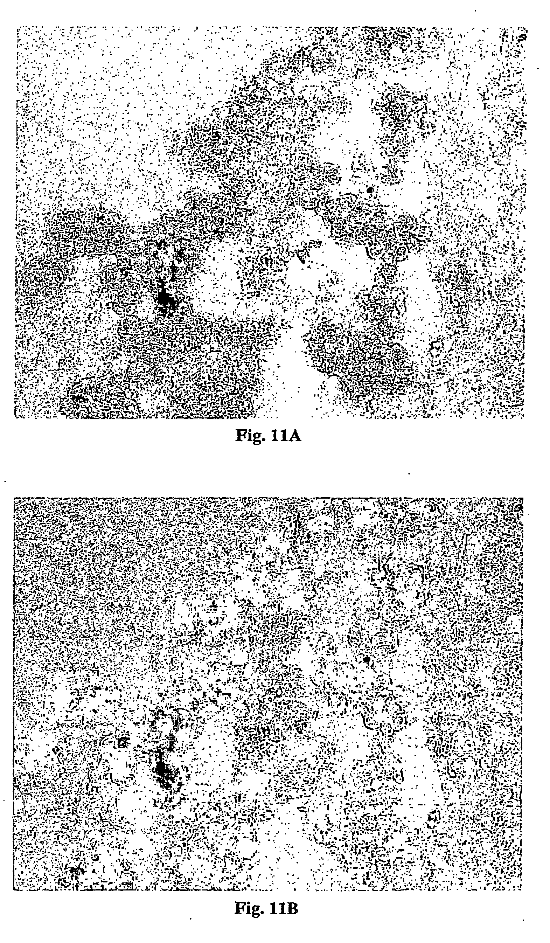

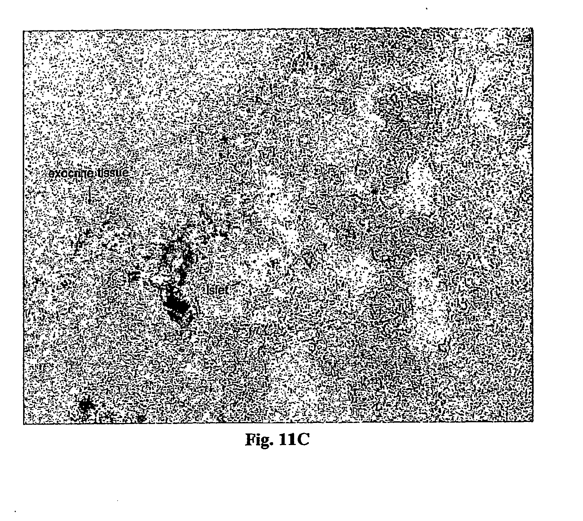

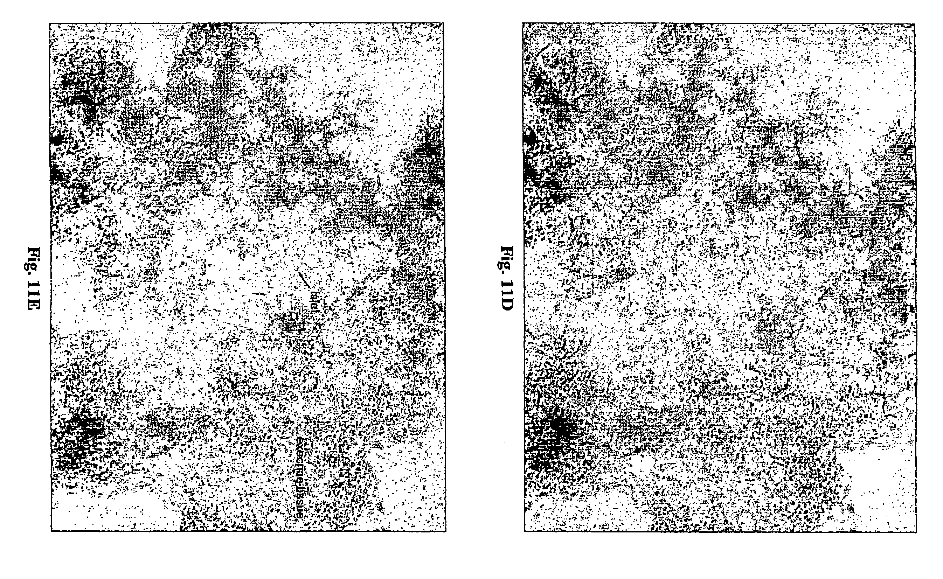

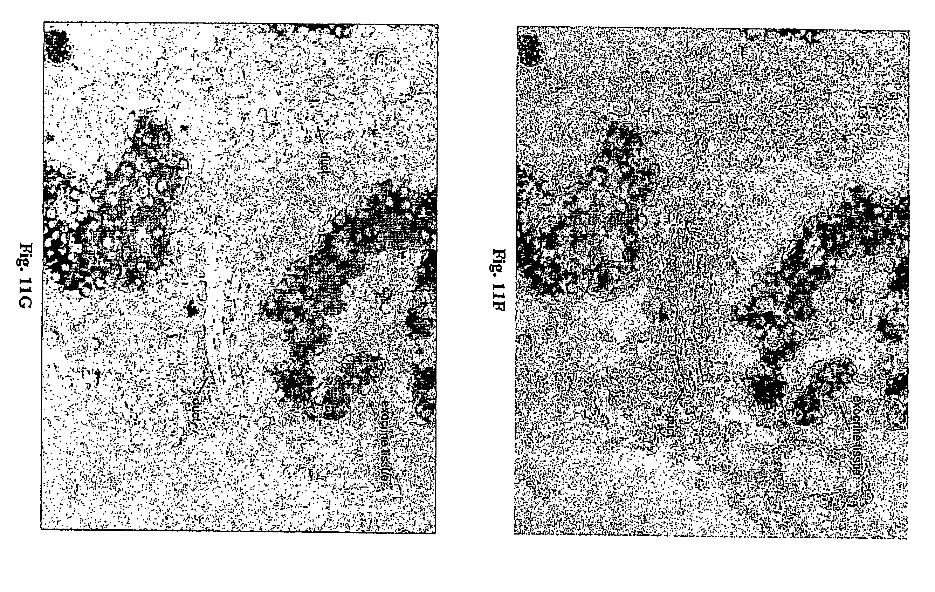

Further, we show sectional in situ hybridizations of SF1 (see Example 5 and Fig. 11). The nucleic acid sequence encoding the mouse SF1 protein is expressed in the pancreas tissue (see Fig. 11). SF1 showed widespread expression in non-beta cell and non-duct cells. The distribution and shape of the expressing cells as well as co-staining with the exocrine marker amylase indicate that SF1 is expressed predominantly in exocrine cells in the developing pancreas.

The invention also encompasses novel use of polynucleotides that encode the proteins for use according to the invention. Accordingly, any nucleic acid sequence, which encodes the amino acid sequences of the proteins for use according to the invention, can be used to generate recombinant molecules that express the proteins of the invention. In a particular embodiment, the invention encompasses a nucleic acid encoding SF6. It will be appreciated by those skilled in the art that as a result of the degeneracy of the genetic code, a multitude of nucleotide sequences encoding the proteins, some bearing minimal homology to the nucleotide sequences of any known and naturally occurring gene, may be produced. The invention contemplates each and every possible variation of nucleotide sequence that can be made by selecting combinations based on possible codon choices.

Also encompassed by the invention is the use of polynucleotide sequences that are capable of hybridizing to the claimed nucleotide sequences, and in particular, those of the polynucleotide encoding the proteins of the invention, under stringent conditions, i. e. that after washing for 1 h with 1 x SSC and 0.1% SDS at 50°C, a positive hybridization signal is observed. Hybridization conditions are based on the melting temperature (Tm) of the nucleic acid binding complex or probe, as taught in Wahl G.M. et al., (1987; Methods Enzymol. 152: 399-407) and Kimmel A.R. (1987; Methods Enzymol. 152: 507-511), and may be used at a defined stringency.

Altered nucleic acid sequences encoding the proteins which are encompassed by the invention include deletions, insertions or substitutions of different nucleotides resulting in a polynucleotide that encodes the same or a functionally equivalent protein.

The encoded proteins may also contain deletions, insertions or substitutions of amino acid residues, which produce a silent change and result in functionally equivalent proteins. Deliberate amino acid substitutions may be made on the basis of similarity in polarity, charge, solubility, hydrophobicity, hydrophilicity, and/or the amphipathic nature of the residues as long as the biological activity of the protein is retained. Also described are peptide fragments of the proteins or derivatives thereof such as cyclic peptides, retro-inverso peptides or peptide mimetics having a length of at least 4, preferably at least 6 and up to 50 amino acids.

Also included in the present disclosure are alleles of the genes encoding the proteins of the invention. As used herein, an 'allele' or 'allelic sequence' is an alternative form of the gene, which may result from at least one mutation in the nucleic acid sequence. Alleles may result in altered mRNAs or polypeptides whose structures or function may or may not be altered. Any given gene may have none, one or many allelic forms. Common mutational changes, which give rise to alleles, are generally ascribed to natural deletions, additions or substitutions of nucleotides. Each of these types of changes may occur alone or in combination with the others, one or more times in a given sequence.

The nucleic acid sequences encoding SF6 may be extended utilizing a partial nucleotide sequence and employing various methods known in the art to detect upstream sequences such as promoters and regulatory elements.

In order to express a biologically active protein, the nucleotide sequences encoding the proteins or functional equivalents, may be inserted into appropriate expression vectors, i.e., a vector which contains the necessary elements for the transcription and translation of the inserted coding sequence. Methods, which are well known to those skilled in the art, may be used to construct expression vectors containing sequences encoding the proteins and the appropriate transcriptional and translational control elements. Regulatory elements include for example a promoter, an initiation codon, a stop codon, a mRNA stability regulatory element, and a polyadenylation signal. Expression of a polynucleotide can be assured by (i) constitutive promoters such as the Cytomegalovirus (CMV) promoter/enhancer region, (ii) tissue specific promoters such as the insulin promoter (see, Soria B. et al., (2000), Diabetes 49: 157-162), SOX2 gene promoter (see Li M. et al., (1998) Curr. Biol. 8: 971-974), Msi-1 promoter (see Sakakibara S. and Okano H., (1997) J. Neuroscience 17: 8300-8312), alpha-cardia myosin heavy chain promoter or human atrial natriuretic factor promoter (Klug M.G. et al., (1996) J. Clin. Invest 98: 216-224; Wu J. et al., (1989) J. Biol. Chem. 264: 6472-6479) or (iii) inducible promoters such as the tetracycline inducible system. Expression vectors can also contain a selection agent or marker gene that confers antibiotic resistance such as the neomycin, hygromycin or puromycin resistance genes. These methods include in vitro recombinant DNA techniques, synthetic techniques, and in vivo genetic recombination. Such techniques are described in Sambrook J. et al., (1989) Molecular Cloning, A Laboratory Manual, Cold Spring Harbor Press, Plainview, N.Y. and Ausubel F.M. et al., (1989) Current Protocols in Molecular Biology, John Wiley & Sons, New York, N.Y.

Also disclosed herein is that natural, modified or recombinant nucleic acid sequences encoding the proteins of the invention may be ligated to a heterologous sequence to encode a fusion protein.

A variety of expression vector/host systems, as known in the art, may be utilized to contain and express sequences encoding the proteins or fusion proteins. These include, but are not limited to, micro-organisms such as bacteria transformed with recombinant bacteriophage, plasmid or cosmid DNA expression vectors; yeast transformed with yeast expression vectors; insect cell systems infected with virus expression vectors (e.g., baculovirus, adenovirus, adeno-associated virus, lentiverus, retrovirus); plant cell systems transformed with virus expression vectors (e.g., cauliflower mosaic virus, CaMV; tobacco mosaic virus, TMV) or with bacterial expression vectors (e.g., Ti or PBR322 plasmids); or animal cell systems.

The presence of polynucleotide sequences of the invention in a sample can be detected by DNA-DNA or DNA-RNA hybridization and/or amplification using probes or portions or fragments of said polynucleotides. Nucleic acid amplification based assays involve the use of oligonucleotides or oligomers based on the sequences specific for the gene to detect transformants containing DNA or RNA encoding the corresponding protein. As used herein 'oligonucleotides' or 'oligomers' refer to a nucleic acid sequence of at least about 10 nucleotides and as many as about 60 nucleotides, preferably about 15 to 30 nucleotides, and more preferably about 20-25 nucleotides, which can be used as a probe or amplimer.

A wide variety of labels and conjugation techniques are known by those skilled in the art and may be used in various nucleic acid and amino acid assays. Means for producing labeled hybridization or PCR probes for detecting polynucleotide sequences include oligo-labeling, nick translation, end-labeling of RNA probes, PCR amplification using a labeled nucleotide, or enzymatic synthesis. These procedures may be conducted using a variety of commercially available kits (Pharmacia & Upjohn, (Kalamazoo, Mich.); Promega (Madison Wis.); and U.S. Biochemical Corp., (Cleveland, Ohio).

The presence of SF1-SF8 in a sample can be determined by immunological methods or activity measurement. A variety of protocols for detecting and measuring the expression of proteins, using either polyclonal or monoclonal antibodies specific for the protein or reagents for determining protein activity are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on the protein is preferred, but a competitive binding assay may be employed. These and other assays are described, among other places, in Hampton R. et al. (1990; Serological Methods, a Laboratory Manual, APS Press, St Paul, Minn.) and Maddox D.E. et al. (1983; J. Exp. Med. 158: 1211-1226).

Suitable reporter molecules or labels, which may be used, include radionuclides, enzymes, fluorescent, chemiluminescent or chromogenic agents as well as substrates, co-factors, inhibitors, magnetic particles, and the like.

The nucleic acids encoding the proteins of the invention can be used to generate transgenic animal or site specific gene modifications in cell lines.

Transgenic animals may be made through homologous recombination, where the normal locus of the genes encoding the proteins of the invention is altered. Alternatively, a nucleic acid construct is randomly integrated into the genome. Vectors for stable integration include plasmids, retroviruses and other animal viruses, YACs, and the like. The modified cells or animal are useful in the study of the function and regulation of the proteins of the invention. For example, a series of small deletions and/or substitutions may be made in the genes that encode the proteins of the invention to determine the role of particular domains of the protein, functions in pancreatic differentiation, etc.

Specific constructs of interest include anti-sense molecules, which will block the expression of the proteins of the invention, or expression of dominant negative mutations. A detectable marker, such as for example lac-Z, may be introduced in the locus of the genes of the invention, where upregulation of expression of the genes of the invention will result in an easily detected change in phenotype.

One may also provide for expression of the genes of the invention or variants thereof in cells or tissues where it is not normally expressed or at abnormal times of development. In addition, by providing expression of the proteins of the invention in cells in which they are not normally produced, one can induce changes in cell behavior.

DNA constructs for homologous recombination will comprise at least portions of the genes of the invention with the desired genetic modification, and will include regions of homology to the target locus. DNA constructs for random integration need not include regions of homology to mediate recombination. Conveniently, markers for positive and/or negative selection are included. Methods for generating cells having targeted gene modifications through homologous recombination are known in the art. For non-humanembryonic stem (ES) cells, an ES cell line may be employed, or embryonic cells may be obtained freshly from a host, e.g. mouse, rat, guinea pig etc. Such cells are grown on an appropriate fibroblast-feeder layer or grown in presence of leukemia inhibiting factor (LIF).

The data disclosed herein show that the SF1-SF8 nucleic acids and proteins and effector/modulator molecules thereof are useful in diagnostic and therapeutic applications implicated, for example, but not limited to, pancreatic diseases (e.g. diabetes, such as insulin dependent diabetes mellitus and/or non insulin dependent diabetes mellitus and/or LADA), obesity, metabolic syndrome, eating disorder, cachexia, hypertension, coronary heart disease, hypercholesterolemia (dyslipidemia), and/or gallstones. Further, the data show that the SF1-SF8 nucleic acids and proteins and effector/modulator molecules thereof are useful for the modulation, e.g. stimulation of pancreatic development, and/or for the regeneration of pancreatic cells or tissues, e.g. cells having exocrinous functions such as acinar cells, centroacinar cells and/or ductal cells and/or cells having endocrinous functions, particularly cells in Langerhans islets such as alpha-, beta-, delta- and/or PP-cells, more particularly beta-cells. Hence, diagnostic and therapeutic uses for the proteins of the invention nucleic acids and proteins of the invention are, for example but not limited to, the following: (i) tissue regeneration in vitro and in vivo (regeneration for all these tissues and cell types composing these tissues and cell types derived from these tissues), (ii) small molecule drug target, (iii) antibody target (therapeutic, diagnostic, drug targeting/cytotoxic antibody), (iv) diagnostic and/or prognostic marker, (v) protein therapy, (vi) gene therapy (gene delivery/gene ablation), and / or (vii) research tools.

For example, but not limited to, cDNAs encoding the proteins for use according to the invention and particularly their human homologues may be useful in stimulating, enhancing or regulating the regeneration of tissues, and the proteins of the invention and particularly their human homologues may be useful when administered to a subject in need thereof. The compositions will have efficacy for treatment of patients suffering from diabetes and/or obesity.

In one embodiment administration of SF6 nucleic acids and proteins and/or effectors/modulators thereof in a pharmaceutical composition to a subject in need thereof, particularly a human patient leads to an at least partial regeneration of, for example, pancreas cells, e.g. endocrine cells and/or exocrine cells. The composition will then at least partially restore normal pancreatic function. In one example, these cells are beta cells of the islets or exocrine cells which will contribute to the improvement of a diabetic state. After administration of this composition on a short term or regular basis, an increase in cell mass can be achieved. This effect upon the body reverses the condition of diabetes partially or completely. As the subject's blood sugar level improves, the dosage administered may be reduced in strength. In at least some cases further administration can be discontinued entirely and the subject continues to produce a normal amount of insulin without further treatment. The subject is thereby not only treated but cured entirely of a diabetic condition. However, even moderate improvements in cell mass can lead to a reduced requirement for exogenous insulin, improved glycemic control and a subsequent reduction in diabetic complications. In another example, other cells of the pancreas can be regenerated in vivo and/or in vitro to cure a certain condition. Beside diabetes, the compositions of the present invention will also have efficacy for treatment of patients with other pancreatic diseases such as pancreatic cancer, dysplasia, or pancreatitis.

The SF6 nucleic acids and proteins and effectors/modulators thereof are useful in diagnostic and therapeutic applications implicated in various embodiments as described below. For example, but not limited to, cDNAs encoding the proteins of the invention and particularly their human homologues may be useful in gene therapy, and the proteins of the invention and particularly their human homologues may be useful when administered to a subject in need thereof. The compositions of the present invention will have efficacy for treatment of patients suffering from diabetes and/or obesity.

The SF6 nucleic acids and proteins and effectors/modulators thereof may be administered either as a monotherapy or as a combination therapy with other pharmaceutical agents. For example, they may be administered together with other pharmaceutical agents suitable for the treatment or prevention of pancreatic diseases and/or obesity. Further, they may be administered together with pharmaceutical agents which have an immunosuppressive activity, e.g. antibodies, polypeptides and/or peptidic or non-peptidic low molecular weight substances. Preferred examples of immunosuppressive agents are listed in the following Table 1.

**Table 1: Exemplary agents for immune suppression**

| **Names** | **Mechanism** |
|---|---|
| 2-amino-1,3-propanediol derivatives | Used for preventing or treating chronic rejection in a patient receiving an organ or tissue allo- or xenotransplant |
| 2-amino-2[2-(4-octylphenyl)ethyl] propane-1,3-diol hydrochloride | Immunosuppression, from accelerated lymphocyte homing |
| 40-O-(2-hydroxyethyl)-rapamycin, SDZ-RAD, Everolimus | Sirolimus (rapamycin) derivative, used for acute kidney rejection; reduces rejection and graft vasculopathy following heart transplantation by inhibiting cell proliferation |
| 6-(3-dimethyl- aminopropionyl) forskolin | Immunosuppressing action useful also for treating autoimmune disease |
| 6-mercaptopurine (6-MP) | Used to treat Crohn's disease, inflammatory bowel disease and for organ transplant therapy |
| A-420983 | Lck-inhibitor |
| ABX-CBL (CBL-1) | Mouse monoclonal AB targeted against human T-cell, B cells, NK cells and monocytes, for treatment of steroid-resistant graft vs host diseases, potential use in treatment of inflammatory and autoimmune disorders |
| Alefacept (human LFA-3 IgGl fusion protein) | Knocks out causative memory T-lymphocytes; Used to treat psoriasis, a T-cell mediated inflammatory disorder |
| Antisense ICAM-1 inhibitor (ISIS 2302), Enlimomab, BIRR1, Alicaforsen | Mouse monoclonal AB blocks white blood cell adhesion to T-cell surface molecule (ICAM-1r); treatment of kidney transplant rejection |
| Antithymocyte immunoglobulin (ATGAM) | Anti-human thymocyte, immunoglobulin; used in reversal of acute kidney transplant rejection and will likely be used off-label for transplant induction therapy |
| Azathioprine | Treatment of rheumatoid arthritis and prevention of kidney transplant rejection, and other autoimmune or inflammatory disorders such as inflammatory bowel disease |
| Baohuoside-1 | Flavonoid; inhibits lymphocyte activation; Ma et al., Transplantation 78: 831-838, (2004) |
| basiliximab | Monoclonal AB that binds to receptor sites on T-cells, preventing activation by transplanted tissue (renal transplant) |
| BMS-279700 | Lck-inhibitor |
| BTI-322 | Mouse derived monoclonal AB targeted to CD2 receptor; used for prevention of first-time kidney rejection, and treatment of resistant rejection |
| Cladribine | Antimetabolite and immunosuppressive agent that is relatively selective for lymphocytes; used to treat lymphoid malignancies, e.g., hairycell leukemia |
| CP-690550 | JAK-3 inhibitor |
| Cyclophosphamide (CTX) | Immunosuppressant for treatment of arthritis and other auto-immune disorders and cancers |
| Cyclosporine (cyclosporin A, cyclosporin) | 11 amino acid cyclic peptide; blocks helper T-cell, immunosuppressant used in organ transplant therapy and other immune diseases |
| Daclizumab, HAT (Humanized Anti-Tac), SMART anti-Tac, anti-CD25, and humanized anti-IL2-receptor | Monoclonal AB inhibits binding of IL-2 to IL-2 receptor by binding to IL-2 receptor, suppresses T-cell activity against allografts (renal transplant) |
| Dexamethasone (Decadron, Dexone, Dexasone) | An adrenocorticoid, effective immunosuppressant in various disorders |
| DIAPEP-277 | Immunomodulatory properties |
| Dipeptide Boronic Acid (DPBA) | Proteasome inhibitor; Wu et al., Transplantation 78: 360-366, (2004) |
| Docosahexaenoic acid (DHA) | Immunosuppressant that lowers the proportion of T cells expressing CD4 or CD8, blocks antigen recognition process; Taku et al., Journal of Agricultural and Food Chemistry 48: 1047, (2000) |
| efalizumab | T-cell modulator that target T-cells through interactions with adhesion molecules on endothelial cell surface, target migration of T-cells into the skin and target activation of T-cells; Used to treat Psoriasis |
| Efomycine M | Leukocyte adhesion inhibitor, Anti-inflammatory |
| FTY720 (oral myriocin derivative) | Alters lymphocyte infiltration into grafted tissues; used for prevention of organ rejection in kidney transplants |
| Glatiramer acetate (co-polymer-1) | Synthetic peptide copolymer, decoy that mimics structure of myelin so immune cells bind Copaxone instead of myelin; for multiple sclerosis |
| Glial fibrillary acidic protein (GFAP) | Possesses immunosuppressive activities in diabetic animal models; Winer et al., Nature Medicine 9: 198, (2003) |
| Gusperimus (15-deoxyspergualin) | Intravenous immunosuppressant; suppresses production of cytotoxic T-cells, neutrophils and macrophages |
| HLA-B2702 peptide | Human peptide, blocks action of NK cells and T-cell mediated toxicities, used for prevention of first kidney allograft rejection |
| hu1124 (anti-CD11a) | Humanized monoclonal antibody; targets CD11a receptor on surface of T cells to selectively inhibit immune system rejection of transplanted organs |
| hOKT31y(Ala-Ala) | non Fc-binding humanized anti CD3 antibody |
| Infliximab . | Monoclonal AB, binds and inactivates human TNFalpha; used to treat Crohn's disease and rheumatoid arthritis |
| Interferon | Immunomodulatory properties |
| ISAtx247 | Used to treat autoimmune diseases such as rheumatoid arthritis and psoriasis |
| isotretinoin | Immunosuppressant, reduces ability of T cells to proliferate in response to immune challenge. Vergelli et al., Immunopharmacology, 31:191, (1997) |
| L-683,742: also described as 31-desmethoxy-31- hydroxy-L-683,590 | Treatment of autoimmune diseases, infectious diseases and/or prevention of organ transplant rejections |
| Leflunomide (ARAVA) | Antiinflammatory agent |
| Medi-500 (T10B9) | Intravenous monoclonal AB that targets human T-cells; treats acute kidney rejection and graft-vs-host disease |
| Medi-507 | Intravenous humanized AB directed against CD2 T-cell; used to treat corticosteroidresistant graft vs host disease and prevention of kidney rejection |
| Methotrexate | Antimetabolite used to treat Crohn's disease, severe psoriasis, and adult rheumatoid arthritis (and as an anti-cancer drug) |
| Mitoxantrone | Antiproliferative effect on cellular immune system including T-cells, B-cells and macrophages; used to treat hormone-refractory prostate cancer, acute myelogenous leukemia and multiple sclerosis |
| mycophenolate mofetil | Proliferation of T and B lymphocytes by blocking the synthesis of purine nucleotides; used in organ transplant therapy and inflammatory bowel disease |
| OKT4A | Mouse monoclonal AB targeted against human CD4 T cell; used for prevention of kidney transplant rejection when used in combination with other immunosuppressant drugs |
| Muromonab-CD3 | Monoclonal AB that binds to receptor sites on T-cells, preventing activation by transplanted tissue |
| Prednisolone | Corticosteroid, suppresses inflammation associated with transplant rejection |
| Psora-4 | Kv 1.3-blocker |
| Rifampicin | Antibiotic; has immunomodulatory properties |
| Rituximab | CD20 antibody |
| S100β | possesses immunosuppressive activities in diabetic animal models |
| Sirolimus, Rapamycin | Immunosuppressant and potent inhibitor of cytokine (e.g.IL-2)-dependent T-cell proliferation (kidney transplant) |
| Tacrolimus (Prograf; FK-506) | Interferes with IL-2 TCR communication |
| Triptolide | Small molecule; inhibits T-cell activation |

The combination therapy may comprise coadministration of the medicaments during the treatment period and/or separate administration of single medicaments during different time intervals in the treatment period.

The nucleic acids for use according to the invention or fragments thereof, may further be useful in diagnostic applications, wherein the presence or amount of the nucleic acids or the proteins are to be assessed. Further antibodies that bind immunospecifically to the novel substances of the invention may be used in therapeutic or diagnostic methods.

For example, in one aspect, antibodies, which are specific for a protein for use according to the invention, may be used directly as an effector/modulator, e.g. an antagonist or an agonist or indirectly as a targeting or delivery mechanism for bringing a pharmaceutical agent to cells or tissue which express the protein. The antibodies may be generated using methods that are well known in the art. Such antibodies may include, but are not limited to, polyclonal, monoclonal, chimeric single chain, Fab fragments, and fragments produced by a Fab expression library. Neutralising antibodies, (i.e., those which inhibit dimer formation) are especially preferred for therapeutic use.

For the production of antibodies, various hosts including goats, rabbits, rats, mice, humans, and others, may be immunized by injection with the protein or any fragment or oligopeptide thereof, which has immunogenic properties. Depending on the host species, various adjuvants may be used to increase immunological response. It is preferred that the peptides, fragments or oligopeptides used to induce antibodies to the protein have an amino acid sequence consisting of at least five amino acids, and more preferably at least 10 amino acids.

Monoclonal antibodies to the proteins may be prepared using any technique that provides for the production of antibody molecules by continuous cell lines in culture. These include, but are not limited to, the hybridoma technique, the human B-cell hybridoma technique, and the EBV-hybridoma technique (Köhler G. and Milstein C., (1975) Nature 256: 495-497; Kozbor D. et al., (1985) J. Immunol. Methods 81: 31-42; Cote R.J. et al., (1983) Proc. Natl. Acad. Sci. 80: 2026-2030; Cole S.P. et al., (1984) Mol. Cell Biol. 62: 109-120).

In addition, techniques developed for the production of 'chimeric antibodies', the splicing of mouse antibody genes to human antibody genes to obtain a molecule with appropriate antigen specificity and biological activity can be used (Morrison S.L. et al., (1984) Proc. Natl. Acad. Sci. 81: 6851-6855; Neuberger M.S. et al., (1984) Nature 312: 604-608; Takeda S. et al., (1985) Nature 314: 452-454). Alternatively, techniques described for the production of single chain antibodies may be adapted, using methods known in the art, to produce single chain antibodies specific for a protein of the invention. Antibodies with related specificity, but of distinct idiotypic composition, may be generated by chain shuffling from random combinatorial immunoglobulin libraries (Kang A.S. et al., (1991) Proc. Natl. Acad. Sci. 88: 11120-11123). Antibodies may also be produced by inducing in vivo production in the lymphocyte population or by screening recombinant immunoglobulin libraries or panels of highly specific binding reagents as disclosed in the literature (Orlandi R. et al., (1989) Proc. Natl. Acad. Sci. 86: 3833-3837; Winter G. and Milstein C., (1991) Nature 349: 293-299).

Antibody fragments, which contain specific binding sites for the proteins may also be generated. For example, such fragments include, but are not limited to, the F(ab')₂ fragments which can be produced by Pepsin digestion of the antibody molecule and the Fab fragments which can be generated by reducing the disulfide bridges of F(ab')₂ fragments. Alternatively, Fab expression libraries may be constructed to allow rapid and easy identification of monoclonal Fab fragments with the desired specificity (Huse W.D. et al., (1989) Science 246: 1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between the protein and its specific antibody. A two-site, monoclonal-based immunoassay utilizing monoclonal antibodies reactive to two non-interfering protein epitopes is preferred, but a competitive binding assay may also be employed (Maddox, supra).

In another embodiment, the polynucleotides or fragments thereof or nucleic acid effector/modulator molecules such as antisense molecules, aptamers, RNAi molecules or ribozymes may be used for therapeutic purposes. In one aspect, aptamers, i.e. nucleic acid molecules, which are capable of binding to a protein of the invention and modulating its activity, may be generated by a screening and selection procedure involving the use of combinatorial nucleic acid libraries.

In a further aspect, antisense molecules may be used in situations in which it would be desirable to block the transcription of the mRNA. In particular, cells may be transformed with sequences complementary to polynucleotides encoding SF6 or homologous proteins. Thus, antisense molecules may be used to modulate/effect protein activity or to achieve regulation of gene function. Such technology is now well known in the art, and sense or antisense oligomers or larger fragments, can be designed from various locations along the coding or control regions of sequences encoding the proteins. Expression vectors derived from retroviruses, adenovirus, herpes or vaccinia viruses or from various bacterial plasmids may be used for delivery of nucleotide sequences to the targeted organ, tissue or cell population. Methods, which are well known to those skilled in the art, can be used to construct recombinant vectors, which will express antisense molecules complementary to the polynucleotides of the genes encoding the proteins of the invention. These techniques are described both in Sambrook et al. (supra) and in Ausubel et al. (supra). Genes encoding the proteins of the invention can be turned off by transforming a cell or tissue with expression vectors, which express high levels of polynucleotides that encode the proteins of the invention or functional fragments thereof. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

As mentioned above, modifications of gene expression can be obtained by designing antisense molecules, e.g. DNA, RNA or nucleic acid analogues such as PNA, to the control regions of the genes encoding SF6, i.e., the promoters, enhancers, and introns. Oligonucleotides derived from the transcription initiation site, e.g., between positions -10 and +10 from the start site, are preferred. Similarly, inhibition can be achieved using "triple helix" base-pairing methodology. Triple helix pairing is useful because it cause inhibition of the ability of the double helix to open sufficiently for the binding of polymerases, transcription factors or regulatory molecules. Recent therapeutic advances using triplex DNA have been described in the literature (Gee J.E. et al., (1994) Gene 149:109-114; Huber B.E. and Carr B.I., (1994) Molecular and Immunologic Approaches, Futura Publishing Co., Mt. Kisco, N.Y.). The antisense molecules may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. Examples, which may be used, include engineered hammerhead motif ribozyme molecules that can be specifically and efficiently catalyze endonucleolytic cleavage of sequences encoding the proteins of the invention. Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridization with complementary oligonucleotides using ribonuclease protection assays.

Nucleic acid effector/modulator molecules, e.g. antisense molecules and ribozymes may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences. Such DNA sequences may be incorporated into a variety of vectors with suitable RNA polymerase promoters such as T7 or SP6. Alternatively, these cDNA constructs that synthesize antisense RNA constitutively or inducibly can be introduced into cell lines, cells or tissues. RNA molecules may be modified to increase intracellular stability and half-life. Possible modifications include, but are not limited to, the addition of flanking sequences at the 5' and/or 3' ends of the molecule or modifications in the nucleobase, sugar and/or phosphate moieties, e.g. the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection and by liposome injections may be achieved using methods, which are well known in the art. Any of the therapeutic methods described above may be applied to any suitable subject including, for example, mammals such as dogs, cats, cows, horses, rabbits, monkeys, and most preferably, humans.

Also disclosed is the administration of a pharmaceutical composition, in conjunction with a pharmaceutically acceptable carrier, for any of the therapeutic effects discussed above. Such pharmaceutical compositions may consist of SF6 nucleic acids or the proteins, antibodies to the proteins of the invention, mimetics, agonists, antagonists or inhibitors of the proteins of the invention or nucleic acids. The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone or in combination with other agents, drugs or hormones. The pharmaceutical compositions may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual or rectal means.

In addition to the active ingredients, these pharmaceutical compositions may contain suitable pharmaceutically-acceptable carriers comprising excipients and auxiliaries, which facilitate processing of the active compounds into preparations, which can be used pharmaceutically. Further details on techniques for formulation and administration may be found in the latest edition of Remington's Pharmaceutical Sciences (Maack Publishing Co., Easton, Pa.).

Pharmaceutical compositions suitable for use according to the invention include compositions wherein the active ingredients are contained in an effective amount to achieve the intended purpose. The determination of an effective dose is well within the capability of those skilled in the art. For any compounds, the therapeutically effective dose can be estimated initially either in cell culture assays, e.g., of preadipocyte cell lines or in animal models, usually mice, rabbits, dogs or pigs. The animal model may also be used to determine the appropriate concentration range and route of administration. Such information can then be used to determine useful doses and routes for administration in humans. A therapeutically effective dose refers to that amount of active ingredient, for example the SF6 nucleic acids or proteins or fragments thereof or antibodies, which is sufficient for treating a specific condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions, which exhibit large therapeutic indices, are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage from employed, sensitivity of the patient, and the route of administration. The exact dosage will be determined by the practitioner, in light of factors related to the subject that requires treatment. Dosage and administration are adjusted to provide sufficient levels of the active moiety or to maintain the desired effect. Factors, which may be taken into account, include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week or once every two weeks depending on half-life and clearance rate of the particular formulation. Normal dosage amounts may vary from 0.1 to 100,000 microg, up to a total dose of about 1 g, depending upon the route of administration. Guidance as to particular dosages and methods of delivery is provided in the literature and generally available to practitioners in the art. Those skilled in the art employ different formulations for nucleotides than for proteins or their inhibitors. Similarly, delivery of polynucleotides or polypeptides will be specific to particular cells, conditions, locations, etc.

In another embodiment, antibodies which specifically bind to the proteins may be used for the diagnosis of conditions or diseases characterized by or associated with over- or under-expression of the proteins for use according to the invention or in assays to monitor patients being treated with the proteins for use according to the invention, or effectors/modulators thereof, e.g. agonists, antagonists, or inhibitors. Diagnostic assays include methods which utilize the antibody and a label to detect the protein in human body fluids or extracts of cells or tissues. The antibodies may be used with or without modification, and may be labeled by joining them, either covalently or non-covalently, with a reporter molecule. A wide variety of reporter molecules, which are known in the art may be used several of which are described above.

A variety of protocols including ELISA, RIA, and FACS for measuring proteins are known in the art and provide a basis for diagnosing altered or abnormal levels of gene expression. Normal or standard values for gene expression are established by combining body fluids or cell extracts taken from normal mammalian subjects, preferably human, with antibodies to the protein under conditions suitable for complex formation. The amount of standard complex formation may be quantified by various methods, but preferably by photometric means. Quantities of protein expressed in control and disease, samples e.g. from biopsied tissues are compared with the standard values. Deviation between standard and subject values establishes the parameters for diagnosing disease.

In another embodiment, the polynucleotides specific for the SF6 proteins may be used for diagnostic purposes. The polynucleotides, which may be used, include oligonucleotide sequences, antisense RNA and DNA molecules, and PNAs. The polynucleotides may be used to detect and quantitate gene expression in biopsied tissues in which gene expression may be correlated with disease. The diagnostic assay may be used to distinguish between absence, presence, and excess gene expression, and to monitor regulation of protein levels during therapeutic intervention.

In one aspect, hybridization with probes which are capable of detecting polynucleotide sequences, including genomic sequences, encoding the proteins of the invention or closely related molecules, may be used to identify nucleic acid sequences which encode the respective protein. The hybridization probes of the subject invention may be DNA or RNA and are preferably derived from the nucleotide sequence of the polynucleotide encoding the proteins of the invention or from a genomic sequence including promoter, enhancer elements, and introns of the naturally occurring gene. Hybridization probes may be labeled by a variety of reporter groups, for example, radionuclides such as ³²P or ³⁵S or enzymatic labels, such as alkaline phosphatase coupled to the probe via avidin/biotin coupling systems, and the like.

Polynucleotide sequences specific for SF6 proteins or homologous nucleic acids may be used for the diagnosis of conditions or diseases, which are associated with the expression of the proteins. Examples of such diseases include the pancreatic diseases (e.g. diabetes), obesity, metabolic syndrome, and/or others. Polynucleotide sequences specific for the SF6 proteins may also be used to monitor the progress of patients receiving treatment for pancreatic diseases (e.g. diabetes), obesity, and/or metabolic syndrome. The polynucleotide sequences may be used qualitative or quantitative assays, e.g. in Southern or Northern analysis, dot blot or other membrane-based technologies; in PCR technologies; or in dip stick, pin, ELISA or chip assays utilizing fluids or tissues from patient biopsies to detect altered gene expression.

In a particular aspect, the SF6 nucleotide sequences may be useful in assays that detect activation or induction of various metabolic diseases or dysfunctions. The nucleotide sequences may be labeled by standard methods, and added to a fluid or tissue sample from a patient under conditions suitable for the formation of hybridization complexes. After a suitable incubation period, the sample is washed and the signal is quantitated and compared with a standard value. The presence of altered levels of nucleotide sequences encoding the proteins of the invention in the sample indicates the presence of the associated disease. Such assays may also be used to evaluate the efficacy of a particular therapeutic treatment regimen in animal studies, in clinical trials or in monitoring the treatment of an individual patient.

In order to provide a basis for the diagnosis of a disease associated with expression of the SF1-SF8 proteins, a normal or standard profile for expression is established. This may be accomplished by combining body fluids or cell extracts taken from normal subjects, either animal or human, with a sequence or a fragment thereof, which is specific for the nucleic acids encoding the proteins of the invention, under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for disease. Deviation between standard and subject values is used to establish the presence of disease. Once disease is established and a treatment protocol is initiated, hybridization assays may be repeated on a regular basis to evaluate whether the level of expression in the patient begins to approximate that, which is observed in the normal patient. The results obtained from successive assays may be used to show the efficacy of treatment over a period ranging from several days to months.

With respect to pancreatic diseases (e.g. diabetes), obesity, and/or metabolic syndrome, the presence of an unusual amount of transcript in biopsied tissue from an individual may indicate a predisposition for the development of the disease or may provide a means for detecting the disease prior to the appearance of actual clinical symptoms. A more definitive diagnosis of this type may allow health professionals to employ preventative measures or aggressive treatment earlier thereby preventing the development or further progression of the metabolic diseases and disorders.

Additional diagnostic uses for oligonucleotides designed from the sequences encoding the proteins for use according to the invention may involve the use of PCR. Such oligomers may be chemically synthesized, generated enzymatically or produced from a recombinant source. Oligomers will preferably consist of two nucleotide sequences, one with sense orientation (5prime.fwdarw.3prime) and another with antisense (3prime.rarw.5prime), employed under optimized conditions for identification of a specific gene or condition. The same two oligomers, nested sets of oligomers or even a degenerate pool of oligomers may be employed under less stringent conditions for detection and/or quantification of closely related DNA or RNA sequences.

In another embodiment, the nucleic acid sequences may also be used to generate hybridization probes, which are useful for mapping the naturally occurring genomic sequence. The sequences may be mapped to a particular chromosome or to a specific region of the chromosome using well known techniques. Such techniques include FISH, FACS or artificial chromosome constructions, such as yeast artificial chromosomes, bacterial artificial chromosomes, bacterial P1 constructions or single chromosome cDNA libraries as reviewed in Price C.M., (1993) Blood Rev. 7: 127-134, and Trask B.J., (1991) Trends Genet. 7: 149-154. FISH (as described in Verma R.S. and Babu A., (1989) Human Chromosomes: A Manual of Basic Techniques, Pergamon Press, New York, N.Y.). The results may be correlated with other physical chromosome mapping techniques and genetic map data. Examples of genetic map data can be found in the 1994 Genome Issue of Science (265: 1981f). Correlation between the location of the gene encoding the proteins of the invention on a physical chromosomal map and a specific disease or predisposition to a specific disease, may help to delimit the region of DNA associated with that genetic disease.

The nucleotide sequences described herein may be used to detect differences in gene sequences between normal, carrier or affected individuals. An analysis of polymorphisms, e.g. single nucleotide polymorphisms may be carried out. Further, in situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti R.A. et al., (1988) Nature 336: 577-580), any sequences mapping to that area may represent associated or regulatory genes for further investigation. The nucleotide sequences of the subject invention may also be used to detect differences in the chromosomal location due to translocation, inversion, etc. among normal, carrier or affected individuals.

In another embodiment, the proteins for use according to the invention, their catalytic or immunogenic fragments or oligopeptides thereof, an in vitro model, a genetically altered cell or animal, can be used for screening libraries of compounds in any of a variety of drug screening techniques. One can identify effectors, e.g. receptors, enzymes, proteins, ligands, or substrates that bind to, modulate or mimic the action of the SF6 proteins. The protein or fragment thereof employed in such screening may be free in solution, affixed to a solid support, borne on a cell surface, or located intracellulary. The formation of binding complexes, between the SF6 proteins and the agent tested, may be measured. Agents could also, either directly or indirectly, influence the activity of the SF6 proteins.

In addition activity of the SF6 proteins against their physiological substrate(s) or derivatives thereof could be measured in cell-based or cell-free assays. Agents may also interfere with posttranslational modifications of the protein, such as phosphorylation and dephosphorylation, farnesylation, palmitoylation, acetylation, alkylation, ubiquitination, proteolytic processing, subcellular localization and degradation. Moreover, agents could influence the dimerization or oligomerization of the proteins of the invention or, in a heterologous manner, of the proteins of the invention with other proteins, for example, but not exclusively, docking proteins, enzymes, receptors, or translation factors. Agents could also act on the physical interaction of the proteins of this invention with other proteins, which are required for protein function, for example, but not exclusively, their downstream signaling.

Methods for determining protein-protein interaction are well known in the art.

For example binding of a fluorescently labeled peptide derived from the interacting protein to the SF6 protein, or vice versa, could be detected by a change in polarisation. In case that both binding partners, which can be either the full length proteins as well as one binding partner as the full length protein and the other just represented as a peptide are fluorescently labeled, binding could be detected by fluorescence energy transfer (FRET) from one fluorophore to the other. In addition, a variety of commercially available assay principles suitable for detection of protein-protein Interaction are well known In the art, for example but not exclusively AlphaScreen (PerkinElmer) or Scintillation Proximity Assays (SPA) by Amersham. Alternatively, the interaction of the SF6 proteins with cellular proteins could be the basis for a cell-based screening assay, in which both proteins are fluorescently labeled and interaction of both proteins is detected by analysing cotranslocation of both proteins with a cellular imaging reader, as has been developed for example, but not exclusively, by Cellomics or EvotecOAl. In all cases the two or more binding partners can be different proteins with one being the SF6 protein or in case of dimerization and/or oligomerization the SF6 protein itself.

Of particular interest are screening assays for agents that have a low toxicity for mammalian cells. The term "agent" as used herein describes any molecule, e.g. protein or pharmaceutical, with the capability of altering or mimicking the physiological function of one or more of the proteins for use according to the invention. Candidate agents encompass numerous chemical classes, though typically they are organic molecules, preferably small organic compounds having a molecular weight of more than 50 and less than about 2,500 Daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Candidate agents are also found among biomolecules including peptides, saccharides, fatty acids, steroids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Where the screening assay is a binding assay, one or more of the molecules may be joined to a label, where the label can directly or indirectly provide a detectable signal.

Another technique for drug screening, which may be used, provides for high throughput screening of compounds having suitable binding affinity to the protein of interest as described in published PCT application WO84/03564. In this method, as applied to the proteins of the invention large numbers of different small test compounds, e.g. aptamers, peptides, low-molecular weight compounds etc., are provided or synthesized on a solid substrate, such as plastic pins or some other surface. The test compounds are reacted with the proteins or fragments thereof, and washed. Bound proteins are then detected by methods well known in the art. Purified proteins can also be coated directly onto plates for use in the aforementioned drug screening techniques. Alternatively, non-neutralizing antibodies can be used to capture the peptide and immobilize it on a solid support. In another embodiment, one may use competitive drug screening assays in which neutralizing antibodies capable of binding the protein specifically compete with a test compound for binding the protein. In this manner, the antibodies can be used to detect the presence of any peptide, which shares one or more antigenic determinants with the protein.

Compounds that bind SF6 proteins, e.g. antibodies, are useful for the identification or enrichment of cells, which are positive for the expression of the proteins of the invention, from complex cell mixtures. Such cell populations are useful in transplantation, for experimental evaluation, and as source of lineage and cell specific products, including mRNA species useful in identifying genes specifically expressed in these cells, and as target for the identification of factors of molecules that can affect them. Cells expressing the protein for use according to the invention or which have been treated with the protein for use according to the invention are useful in transplantation to provide a recipient with pancreatic islet cells, including insulin producing beta cells; for drug screening; experimental models of islet differentiation and interaction with other cell types; in vitro screening assays to define growth and differentiation factors, and to additionally characterize genes involved in islet development and regulation; and the like.
The native cells may be used for these purposes, or they may be genetically modified to provide altered capabilities. Cells from a regenerating pancreas, from embryonic foregut, stomach and duodenum, or other sources of pancreatic progenitor cells may be used as a starting population. The progenitor cells may be obtained from any mammalian species, e.g. equine, bovine, porcine, canine, feline, rodent, e.g. mice, rats, hamster, primate, etc. particularly human.

In another embodiment, in a high-throughput screening method, the cells are transfected with a DNA construct, e.g. a viral or non-viral vector containing a reporter gene, e.g. the lacZ gene or the GFP gene, under regulatory control of a promoter of a gene involved in for example beta-cell differentiation, e.g. a promoter of a gene stimulation beta-cell differentiation, preferably a Pax4 promoter. The transfected cells are divided into aliquots and each aliquot is contacted with a test substance, e.g., candidate 1, candidate 2 and candidate 3. The activity of the reporter gene corresponds to the capability of the test compound to induce beta-cell differentiation.

In a further embodiment, which may be combined with the high-throughput screening as described above, a medium throughput validation is carried out.
Therein, the test compound is added to stem cells being cultivated and the insulin production is determined. Following an initial high throughput assay, such as the cell based assay outlined above where for example a Pax4 promoter is used as marker for beta-cell regeneration, the activity of candidate molecules to induce beta-cell differentiation is tested in a validation assay comprising adding said compounds to the culture media of the embryoid bodies. Differentiation into insulin-producing cells is then evaluated, e.g. by comparison to wild type and/or Pax4 expressing ES cells to assess the effectiveness of a compound.

The nucleic acids encoding the SF6 proteins can be used to generate transgenic cell lines and animals. These transgenic non-human animals are useful in the study of the function and regulation of the proteins of the invention in vivo. Transgenic animals, particularly mammalian transgenic animals, can serve as a model system for the investigation of many developmental and cellular processes common to humans. A variety of non-human models of metabolic disorders can be used to test modulators of the SF6 protein. Misexpression (for example, overexpression or lack of expression) of the SF6 protein, particular feeding conditions, and/or administration of biologically active compounds can create models of metabolic disorders.

Such assays use mouse models of insulin resistance and/or diabetes, such as mice carrying gene knockouts in the leptin pathway (for example, ob (leptin) or db (leptin receptor) mice), as described above. In addition to testing the expression of the SF6 proteins in such mouse strains (see Examples), these mice could be used to test whether administration of a candidate modulator alters for example lipid accumulation in the liver, in plasma, or adipose tissues using standard assays well known in the art, such as FPLC, colorimetric assays, blood glucose level tests, insulin tolerance tests and others.

Transgenic animals may be made through homologous recombination in embryonic stem cells, where the normal locus of the gene encoding the SF6 protein is mutated. Alternatively, a nucleic acid construct encoding the protein is injected into oocytes and is randomly integrated into the genome. One may also express the genes of the invention or variants thereof in tissues where they are not normally expressed or at abnormal times of development. Furthermore, variants of the genes of the invention like specific constructs expressing anti-sense molecules or expression of dominant negative mutations, which will block or alter the expression of the proteins of the invention may be randomly integrated into the genome. A detectable marker, such as lac Z or luciferase may be introduced into the locus of the genes of the invention, where upregulation of expression of the genes of the invention will result in an easily detectable change in phenotype. Vectors for stable integration include plasmids, retroviruses and other animal viruses, yeast artificial chromosomes (YACs), and the like. DNA constructs for homologous recombination will contain at least portions of the genes of the invention with the desired genetic modification, and will include regions of homology to the target locus. Conveniently, markers for positive and negative selection are included. DNA constructs for random integration do not need to contain regions of homology to mediate recombination. DNA constructs for random integration will consist of the nucleic acids encoding the proteins of the invention, a regulatory element (promoter), an intron and a poly-adenylation signal. Methods for generating cells having targeted gene modifications through homologous recombination are known in the field. For embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF). ES or embryonic cells may be transfected and can then be used to produce transgenic animals. After transfection, the ES cells are plated onto a feeder layer in an appropriate medium. Cells containing the construct may be selected by employing a selection medium. After sufficient time for colonies to grow, they are picked and analyzed for the occurrence of homologous recombination. Colonies that are positive may then be used for embryo manipulation and morula aggregation. Briefly, morulae are obtained from 4 to 6 week old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or in vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the proteins of the invention in vivo.

Finally, the invention also relates to a kit comprising at least one of
(a) a nucleic acid molecule coding for a SF6 protein;
(b) a SF6 protein of the invention or a fragment or an isoform thereof;
(c) a vector comprising the nucleic acid of (a);
(d) a host cell comprising the nucleic acid of (a) or the vector of (c);
(e) a polypeptide encoded by the nucleic acid of (a);
(f) a fusion polypeptide encoded by the nucleic acid of (a);
(g) an antibody, an aptamer or another effector / modulator against the nucleic acid of (a) or the polypeptide of (b), (e) or (f) and
(h) an anti-sense oligonucleotide of the nucleic acid of (a).

The kit may be used for diagnostic or therapeutic purposes or for screening applications as described above. The kit may further contain user instructions.

The Figures show:
**Fig. 1** shows the analysis of SF1 expression in mammalian (mouse) tissues.
**Fig. 1A** shows the real-time PCR analysis of SF1 expression in wild type mouse tissues (referred to as wt-mice) and in tissues of mice fed with a control diet (referred to as controldiet).
**Fig. 1B** shows the real-time PCR analysis of SF1 expression in genetically obese mice (referred to as ob/ob-mice) compared to wild type mice, and in mice fed with a high fat diet (referred to as HFD-mice) compared to mice fed with a control diet.
**Fig. 2** shows the analysis of SF2 expression in mammalian (mouse) tissues.
**Fig. 2A** shows the real-time PCR analysis of SF2 expression in wild type mouse tissues (referred to as wt-mice) and in tissues of mice fed with a control diet (referred to as controldiet).
**Fig. 2B** shows the real-time PCR analysis of SF2 expression in genetically obese mice (referred to as ob/ob-mice) compared to wild type mice, and in mice fed with a high fat diet (referred to as HFD-mice) compared to mice fed with a control diet.
**Fig. 3** shows the analysis of SF3 expression in mammalian (mouse) tissues.
**Fig. 3A** shows the real-time PCR analysis of SF3 expression in wild type mouse tissues (referred to as wt-mice) and in tissues of mice fed with a control diet (referred to as controldiet).
**Fig. 3B** shows the real-time PCR analysis of SF3 expression in genetically obese mice (referred to as ob/ob-mice) compared to wild-type mice, and in mice fed with a high fat diet (referred to as HFD-mice) compared to mice fed with a control diet.
**Fig. 4** shows the analysis of SF4 expression in mammalian (mouse) tissues.
**Fig. 4A** shows real-time PCR the analysis of SF4 expression in wild type mouse tissues (referred to as wt-mice) and in tissues of mice fed with a control diet (referred to as controldiet).
**Fig. 4B** shows the real-time PCR analysis of SF4 expression in fasted mice (referred to as fasted-mice) and genetically obese mice (referred to as ob/ob-mice) compared to wild-type mice, and in mice fed with a high fat diet (referred to as HFD-mice) compared to mice fed with a control diet.
**Fig. 4C** shows the real-time PCR analysis of SF4 expression in mouse tissues from non-obese-diabetic (NOD) mice compared to wild-type mice.
**Fig. 5** shows the analysis of SF5 expression in mammalian (mouse) tissues.
**Fig. 5A** shows the real-time PCR analysis of SF5 expression in wild type mouse tissues (referred to as wt-mice) and in tissues of mice fed with a control diet (referred to as controldiet).
**Fig. 5B** shows the real-time PCR analysis of SF5 expression in genetically obese mice (referred to as ob/ob-mice) compared to wild type mice, and in mice fed with a high fat diet (referred to as HFD-mice) compared to mice fed with a control diet.
**Fig. 6** shows the analysis of SF6 expression in mammalian (mouse) tissues.
**Fig. 6A** shows the real-time PCR analysis of SF6 expression in wild type mouse tissues (referred to as wt-mice) and in tissues of mice fed with a control diet (referred to as controldiet).
**Fig. 6B** shows the real-time PCR analysis of SF6 expression in tissues of mice fed with a control diet (without brain).
**Fig. 6C** shows the real-time PCR analysis of SF6 expression in genetically obese mice (referred to as ob/ob-mice) compared to wild type mice, and in mice fed with a high fat diet (referred to as HFD-mice) compared to mice fed with a control diet.
**Fig. 7** shows the analysis of SF7 expression in mammalian (mouse) tissues.
**Fig. 7A** shows the real-time PCR analysis of SF7 expression in wild type mouse tissues (referred to as wt-mice) and in tissues of mice fed with a control diet (referred to as controldiet).
**Fig. 7B** shows the real-time PCR analysis of SF7 expression in genetically obese mice (referred to as ob/ob-mice) compared to wild type mice, and in mice fed with a high fat diet (referred to as HFD-mice) compared to mice fed with a control diet.
**Fig. 8** shows the analysis of SF8 expression in mammalian (mouse) tissues.
**Fig. 8A** shows the real-time PCR analysis of SF8 expression in wild type mouse tissues (referred to as wt-mice) and in tissues of mice fed with a control diet (referred to as controldiet).
**Fig. 8B** shows the real-time PCR analysis of SF8 expression in genetically obese mice (referred to as ob/ob-mice) compared to wild type mice, and in mice fed with a high fat diet (referred to as HFD-mice) compared to mice fed with a control diet.
**Fig. 9** shows the microarray analysis of SF3 expression in human abdominal adipocyte cells during the differentiation from preadipocytes to mature adipocytes.
**Fig. 10A** shows the microarray analysis of SF5 expression in human abdominal adipocyte cells during the differentiation from preadipocytes to mature adipocytes.
**Fig. 10B** shows the microarray analysis of SF5 expression in human SGBS cells during the differentiation from preadipocytes to mature adipocytes.
**Fig. 11** shows in situ hybridization results for the SF1 protein of the invention.
**Fig. 11A** shows a cryosection of embryonic mouse pancreas (day E17.5),
stained with an in situ hybridizaton method.
**Fig. 11B** shows a cryosection of embryonic mouse pancreas (day E17.5), stained with an in situ hybridizaton method and co-stained with the exocrine marker amylase (red).
**Fig. 11C** shows a cryosection of embryonic mouse pancreas (day E17.5), stained with an in situ hybridizaton method and co-stained with the beta cell marker insulin (green).
**Fig. 11D** shows a cryosection of embryonic mouse pancreas (day E17.5), stained with an in situ hybridizaton method, and co-stained with the exocrine marker amylase (red).
**Fig. 11E** shows a cryosection of embryonic mouse pancreas (day E17.5) stained with an in situ hybridizaton method, and co-stained with the beta cell marker insulin (green).
**Fig. 11F** shows a cryosection of embryonic mouse pancreas (day E17.5), stained with an in situ hybridizaton method.
**Fig. 11G** shows a cryosection of embryonic mouse pancreas (day E17.5) stained with an in situ hybridizaton method, and co-stained with the beta cell marker insulin (green) and the duct cell marker DBA lectin (blue).

The examples illustrate the invention:

### Example 1: Identification of secreted factors expressed in pancreas

A screen for secreted factors expressed in developing mouse pancreas was carried out according to methods known by those skilled in the art (see, for example Pera E.M. and De Robertis E.M., (2000) Mech Dev 96(2): 183-195) with several modifications.

### Expression cDNA library:

During organogenesis, the pancreatic bud is surrounded and influenced by the associated mesenchyme. (see for example, Madsen O.D. et al., (1996) Eur. J. Biochem. 242: 435-445 and Slack, J.M., (1995) Development 121: 1569-1580). Recently, it was suggested, that white adipocytes origin directly from mesenchymal cells (Atanossova P.K., (2003) Folia Med. 45: 41-45). During embryogenesis, the innervation and vascularization of the pancreas can be observed. Therefore, the tissue used in the screen might have contained besides pancreatic cells some adipocyte precursors, blood vessels, as well as neuronal cells.

A mouse embryonic stage 9.5-15 pancreatic bud library was prepared in pCMVSPORT-6 vector using SUPERSCRIPT Plasmid System from Invitrogen according to the manufacturer's instructions. The non-amplified library was electroporated into MaxEff DH10B cells (Invitrogen).

Secretion cloning

Bacterial clones were picked with sterile toothpicks from agar plates and cultured in 96-deep-well microtiter plates in LB-ampicillin (see Sambrook et al., supra). Aliquots of 8 cultures were pooled, and plasmid DNA was isolated using the BioRobot_9600 apparatus according to the manufacturer's instructions (Qiagen; QlAprep(r) Turbo BioRobot Kit. Human 293 cell culture cells were cultured in 75 ml tissue culture flasks in DMEM and 10% fetal calf serum. At 90-99% confluence, the cells were splitted at 1:3 ratio and plated onto poly-D-lysine (Sigma) coated 96-well plates. Cells were transfected with 100-500 ng plasmid using lipofectamine 2000 (Invitrogen). After 6 hours, the medium was exchanged for fresh complete growth medium. 24 hours after transfection, the cells were washed twice with DMEM without cysteine and methionine (Invitrogen), supplemented with 1% dialysed bovine serum (Sigma) with 50 µg/ml per ml Heparin (Sigma) and glutamine. The cells were labeled radioactively ('S35 Met-label', from Hartmann Analytic GmbH). After 12 hours, aliquots of the supernatants were harvested in 96-well PCR plates and subjected to SDS gel electrophoresis in precast 4□20% gradient polyacrylamide Criterion gels (Biorad) under reducing conditions, using Criterion Dodeca Cell gel running chamber (Biorad). The gels were fixed in 10% acetic acid, 25% isopropanol for 30 min, soaked 15-30 min in AMPLIFY reagent (Amersham), dried and exposed to X-OMAT (AR) film (Kodak). Positive clones were identified and regrown in 96-well-plates. DNA of individual clones was prepared and used for transfection as described above. If one of the clones yielded proteins of the same size as that of the original pool, a positive clone was identified. Positive clones were partially sequenced from the 5' end (SEQLAB, Goettingen).

### Example 2: Identification of the human homologous nucleic acid and protein sequences

The term "polynucleotide comprising the nucleotide sequence as shown in GenBank Accession number" relates to the expressible gene of the nucleotide sequences deposited under the corresponding GenBank Accession number. The term "GenBank Accession number" relates to NCBI GenBank database entries (Ref.: Benson D.A. et al., (2000) Nucleic Acids Res. 28: 15-18).

SF1-SF8 homologous proteins and nucleic acid molecules coding therefore are obtainable from insect or vertebrate species, e.g. mammals or birds. Particularly preferred are nucleic acids comprising human homologs as described in Table 1. The mouse sequences identfied in the ,secreted factor screen', are also shown in Table 1.

Sequences homologous to the mouse sequences were identified using the publicly available program BLASTP 2.2.3 of the non-redundant protein data base of the National Center for Biotechnology Information (NCBI) (see, Altschul S.F. et al., (1997) Nucleic Acids Res. 25: 3389-3402). The best human homolog of the mouse sequences are shown in Table 2.

**Table 2: Mammalian genes and proteins of the invention (SF1-SF8)**

| **Name** | **Genbank Accession Numbers** | | | |
|---|---|---|---|---|
| | **Mus musculus genes and proteins** | | **Homo sapiens genes and proteins** | |
| | **cDNA** | **Protein** | **cDNA** | **Protein** |
| SF1 | NM 026522 | NP 080798 | NM 023947 | NP 076436 |
| SF2 | NM 023625 | NP 076114 | NM 173542 | NP 775813 |
| SF3 | AS 051390 | BAB18461 | NM 145584 | NP 663559 |
| SF4 | NM 009312 | NP 033338 | NM 013251 | NP 037383 |
| SF5 | AB025280 | BAA76386 | NM 005928 | NP 005919 |
| SF6 | NM 016743 | NP 058023 | NM 006159 | NP 006150 |
| SF7 | NM 007618 | NP 031644 | NM 001756 | NP 001747 |
| SF8 | NM 008908 | NP 032934 | NM 000943 | NP 000934 |

### Example 3: Taqman analysis of the expression of the SF1-SF8 nucleic acids in mammalian (mouse) tissues

To analyse the expression of the SF1-SF8 mRNAs disclosed in this invention in mammalian tissues, several mouse strains (preferably mice strains C57Bl/6J, C57Bl/6 ob/ob, C57Bl/KS db/db, and Non-Obese-Diabetic (NOD) mice, which are standard model systems in obesity and diabetes research) were purchased from Harlan Winkelmann (33178 Borchen, Germany) and Taconic M & B (Germantown, NY 12526, U.S.A.), respectively, and maintained under constant temperature (preferably 22°C), 40 per cent humidity and a light / dark cycle of preferably 14 / 10 hours. The mice were fed a standard chow (for example, from ssniff Spezialitäten GmbH, order number ssniff M-Z V1126-000). For the fasting experiment ("fasted wild type mice"), wild type mice were starved for 48 h without food, but only water supplied ad libitum. (see, for example, Schnetzler et al. J Clin Invest 1993 Jul;92(1):272-80, Mizuno et al. Proc Natl Acad Sci U S A 1996 Apr 16;93(8):3434-8). In a further experiment wild-type (wt) mice were fed a control diet (preferably Altromin C1057 mod control, 4.5% crude fat) or high fat diet (preferably Altromin C1057mod. high fat, 23.5% crude fat). Animals were sacrificed at an age of 6 to 8 weeks. The animal tissues were isolated according to standard procedures known to those skilled in the art, snap frozen in liquid nitrogen and stored at -80°C until needed.

RNA was isolated from mouse tissues using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferably using Superscript II RNaseH- Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

The following prime/probe pairs were used for the TaqMan analysis (GenBank Accession Number NM_026522 (mouse) for the mouse SF1 sequence):
Mouse SF1 forward primer (Seq ID NO:1): 5'- GTT GCC AAG GTC TTT GGG AG -3'; mouse SF1 reverse primer (Seq ID NO:2): 5'- CTC ACG ACC ACG TCT CTT CAG T -3'; mouse SF1 Taqman probe (Seq ID NO:3): (5/6-FAM)- AGT TCA CAC AGA TCT CAC CAG TCT GGT TGC -(5/6-TAMRA).

The following prime/probe pairs were used for the TaqMan analysis (GenBank Accession Number NM_023625 (mouse) for the mouse SF2 sequence):

Mouse SF2 forward primer (Seq ID NO:4): 5'- GCC ATG GTC CGG CTC A
- 3'; mouse SF2 reverse primer (Seq ID NO:5): 5'- CTT TGG GTT GCA GGC TTC A -3'; mouse SF2 Taqman probe (Seq ID NO:6): (5/6-FAM)- TGA TTT CCT CCA TGA CCC TCT GTC ATT GT -(5/6-TAMRA).

The following prime/probe pairs were used for the TaqMan analysis (GenBank Accession Number AB051390 (mouse) for the mouse SF3 sequence):

Mouse SF3 forward primer (Seq ID NO:7): 5'- CCG GGA GAG ATA CGT GAA GC -3'; mouse SF3 reverse primer (Seq ID NO:8): 5'- TCT CCG TCT CCT CCG TGG -3'; mouse SF3 Taqman probe (Seq ID NO:9): (5/6-FAM)-CCG GAA GAC GGC TCA GTG TGC AT -(5/6-TAMRA).

The following prime/probe pairs were used for the TaqMan analysis (GenBank Accession Number NM_009312 (mouse) for the mouse SF4 sequence):

Mouse SF4 forward primer (Seq ID NO:10): 5'- AGC TGC CTC CGT CCC TG -3'; mouse SF4 reverse primer (Seq ID NO:11): 5'- TTC AGC AAT CCT TCC AGA GAG AC-3'; mouse SF4 Taqman probe (Seq ID NO:12): (5/6-FAM)- TTC GGA GAC TCT ACG ACA GCC GCC-(5/6-TAMRA).

The following prime/probe pairs were used for the TaqMan analysis (GenBank Accession Number AB025280 (mouse) for the mouse SF5 sequence):

Mouse SF5 forward primer (Seq ID NO:13): 5'- CGG GCC AAG ACA ATG ACA TC -3'; mouse SF5 reverse primer (Seq ID NO:14): 5'- GTC TCA TTG CAC ACA AGG CCT -3'; mouse SF5 Taqman probe (Seq ID NO:15): (5/6-FAM)- ACT GCC TCT GCC CTG AAG GCT TCA -(5/6-TAMRA).

The following prime/probe pairs were used for the TaqMan analysis (GenBank Accession Number NM_016743 (mouse) for the mouse SF6 sequence):

Mouse SF6 forward primer (Seq ID NO:16): 5'- TCG CTT CAC CGG GTC TTC -3'; mouse SF6 reverse primer (Seq ID NO:17): 5'- GGC CAT TCT TGC ACT GGC -3'; mouse SF6 Taqman probe (Seq ID NO:18): (5/6-FAM)-ATC AAG CAC GGC ACG GAG TGT ACC C -(5/6-TAMRA).

The following prime/probe pairs were used for the TaqMan analysis (GenBank Accession Number NM_007618 (mouse) for the mouse SF7 sequence):

Mouse SF7 forward primer (Seq ID NO:19): 5'- CCA TGC TGC AAC TGG ATG AA -3'; mouse SF7 reverse primer (Seq ID NO:20): 5'- CAG GTG TAC AGG AGG GCC A -3'; mouse SF7 Taqman probe (Seq ID NO:21): (5/6-FAM)- AAT GTG CTG CCT GCT GCC ACC A -(5/6-TAMRA).

The following prime/probe pairs were used for the TaqMan analysis (GenBank Accession Number NM_008908 (mouse) for the mouse SF8 sequence):

Mouse SF8 forward primer (Seq ID NO:22): 5'- TGG CTG GGT CAG CAT GG -3'; mouse SF8 reverse primer (Seq ID NO:23): 5'- TCA AGG TGA TGA AGA ACT GGG A -3'; mouse SF8 Taqman probe (Seq ID NO:24): (5/6-FAM)- CAA TGC TGG ACC AGA CAC CAA CGG -(5/6-TAMRA).

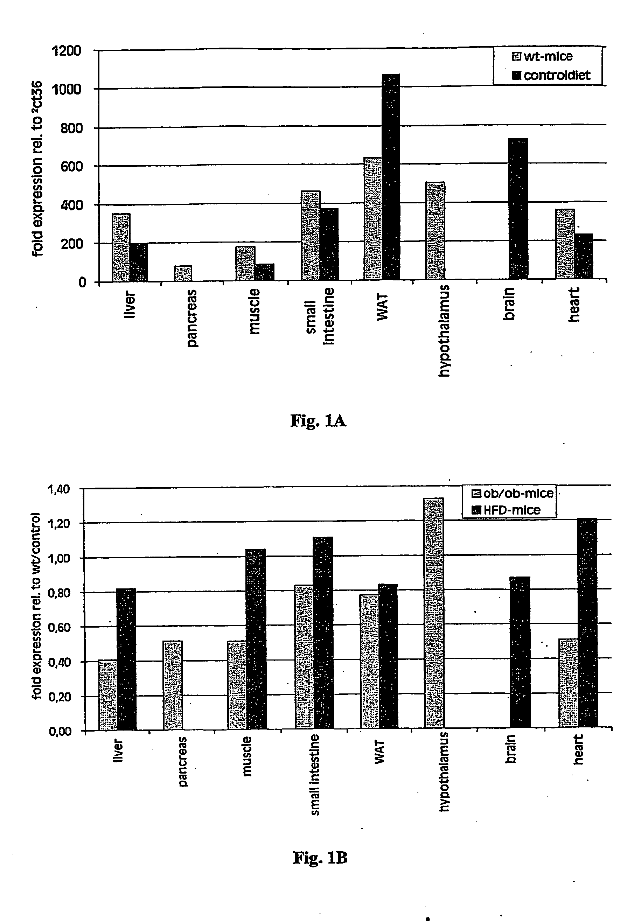

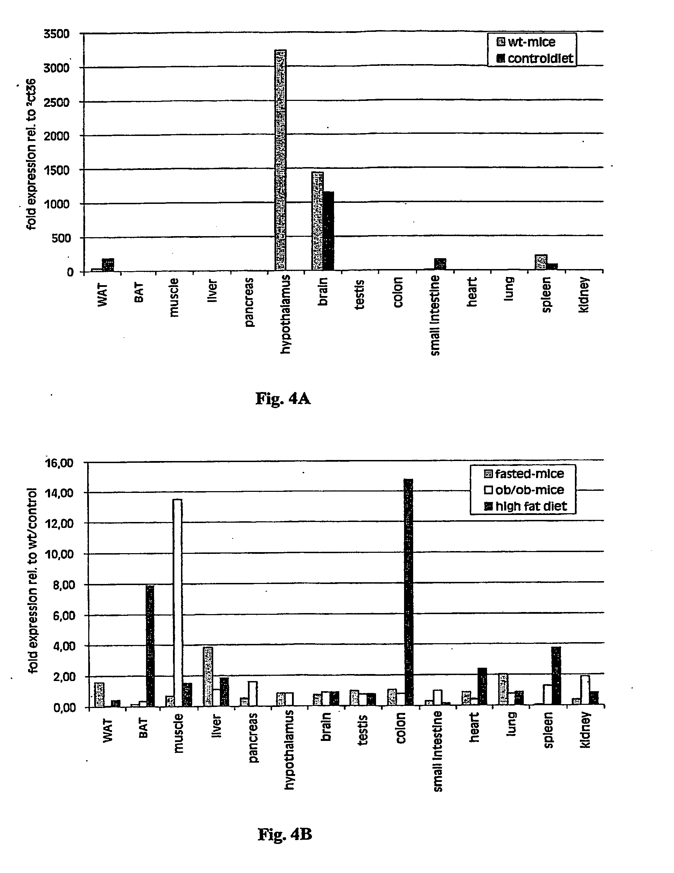

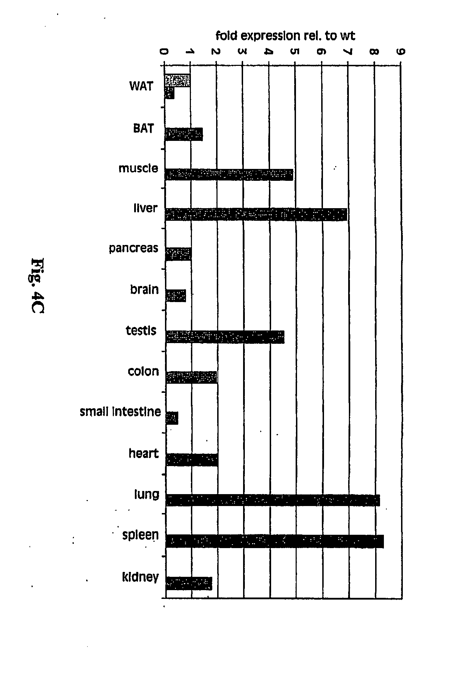

In the figures the relative RNA-expression is shown on the Y-axis. In Fig. 1 2, 3, 4, 5, 6, 7, and 8, the tissues tested are given on the X-axis. "WAT" refers to white adipose tissue, "BAT" refers to brown adipose tissue. In Fig. 1 2, 3, 5, 6, 7, and 8, the panel of the wild type mice tissues comprises liver, pancreas, muscle, small intestine, WAT, hypothalamus, and heart, and the panel of the control diet-mice tissues comprises liver, muscle, small intestine, WAT, brain, and heart. In Fig. 4 the panel of the wild type mice tissues comprises WAT, BAT, muscle, liver, pancreas, hypothalamus, brain, testis, colon, small intestine, heart, lung, spleen, and kidney, and the panel of the control diet-mice tissues comprises WAT, BAT, muscle, liver, brain, testis, colon, small intestine, heart, lung, spleen, and kidney.

The function of the SF1-SF8 proteins in metabolism was further validated by analyzing the expression of the transcripts in different tissues. For this passage, mouse models of insulin resistance and/or diabetes were used, such as mice carrying gene knockouts in the leptin pathway (for example, ob/ob (leptin) or db/db (leptin receptor/ligand) mice) to study the expression of the proteins of the invention. Such mice develop typical symptoms of diabetes, show hepatic lipid accumulation and frequently have increased plasma lipid levels (see Bruning J.C. et al, (1998) Mol. Cell. 2: 559-569).

Further, expression of the mRNAs encoding the SF1-SF8 proteins was also examined in susceptible wild type mice (for example, C57Bl/6) that show symptoms of diabetes, lipid accumulation, and high plasma lipid levels, if fed a high fat diet.

Expression profiling studies confirm the particular relevance of the SF1-SF8 proteins as regulators of energy metabolism in mammals.

Taqman analysis revealed that SF1 is expressed in several mammalian tissues, showing highest level of expression in white adipose tissue (WAT) and brain, and lower but still robust levels in further tissues, e.g. hypothalamus, small intestine, heart, liver, muscle, and pancreas, as shown in Fig. 1A. We found, for example, that the expression of SF1 is down-regulated in the liver, pancreas, muscle, and heart of ob/ob mice compared to wild-type mice (see Fig. 1 B). In wild type mice fed a high fat diet, the expression of SF1 is not regulated. The ubiqitous and robust expresssion levels of SF1 suggest that it plays an essential role in cellular metabolism The regulation of gene expression in the mouse model for the metabolic syndrome as described above suggests that it also might play a role in the regulation of energy homeostasis.

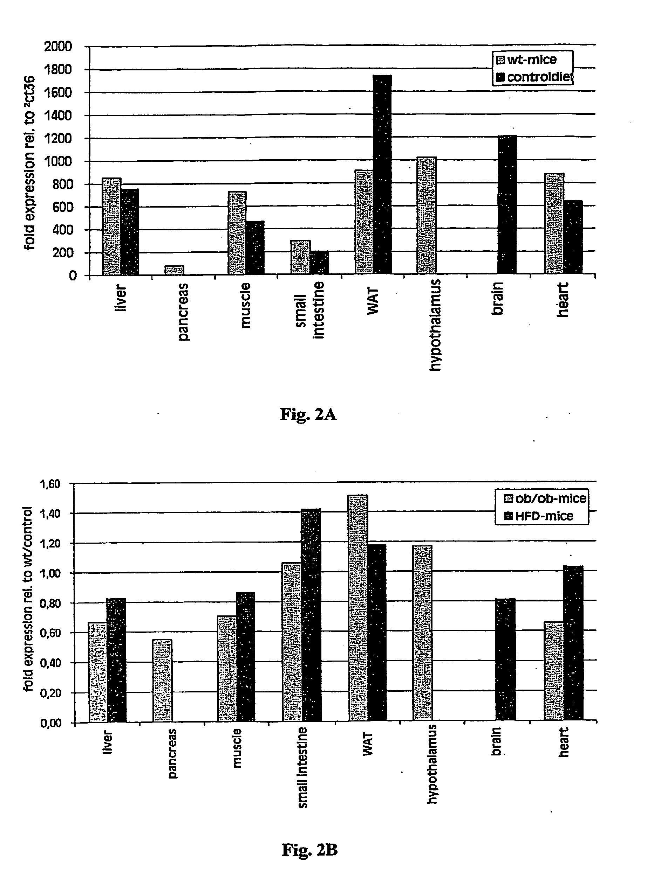

Taqman analysis revealed that SF2 is expressed in several mammalian tissues, showing highest level of expression in WAT, brain, and hypothalamus, and higher levels in further tissues, e.g. liver, heart, and muscle. Furthermore SF2 is expressed on lower but still robust levels in the small intestine and pancreas as depicted in Fig. 2A. We found, for example, that the expression of SF2 is slightly down-regulated in the pancreas of ob/ob mice compared to wild-type mice (see Fig. 2B). In wild type mice fed a high fat diet, the expression of SF2 is not regulated. The ubiqitous and robust expresssion levels of SF2 suggest that it plays an essential role in cellular metabolism The regulation of gene expression in the mouse model for the metabolic syndrome as described above suggests that it also might play a role in the regulation of energy homeostasis.

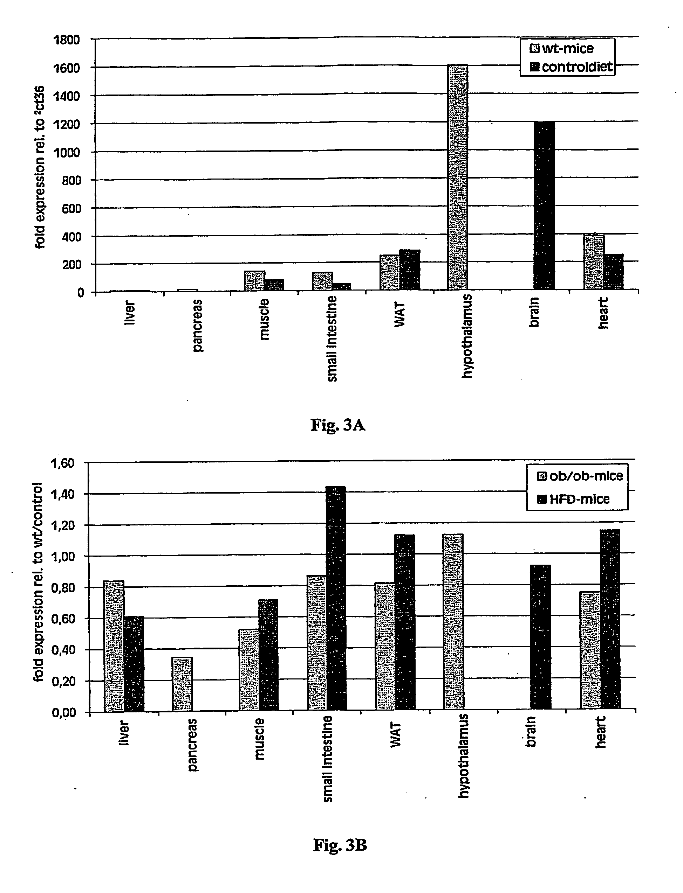

Taqman analysis revealed that SF3 is expressed in several mammalian tissues, showing highest level of expression in brain and hypothalamus, and higher levels in further tissues, e.g. WAT and heart. Furthermore SF3 is expressed on lower but still robust levels in the muscle and small intestine as depicted in Fig. 3A. Even though SF3 in pancreas of wild type mice was expressed on a low level, a clear down-regulation could be observed in the pancreas of ob/ob-mice (Fig. 3B). In wild type mice fed a high fat diet, the expression of SF3 was not regulated. The expresssion of SF3 in metabolic active tissues of wild type and control mice as well as the regulation of gene expression in the mouse model for the metabolic syndrome as described above suggests that it might play a role in the regulation of energy homeostasis.

Taqman analysis revealed that the expression of SF4 is restricted to a few mammalian tissues, with highest expression levels in hypothalamus and brain of wild type mice. In addition, SF4 is expressed in spleen, white adipose tissue (WAT) and at lower levels in small intestine of wild type and control mice as depicted in Fig. 4A. We found, for example, that the expression of SF4 is significantly downregulated in metabolic active tissue (WAT) and upregulated in muscle of genetically induced obese mice (*ob*/*ob*) compared to wild type mice. Further, we found that the expression of SF4 is significantly downregulated in spleen of starved wild type mice compared to wild type mice fed a standard diet (see Fig. 4B). In wild type mice fed a high fat diet, the expression of SF4 is downregulated in WAT and small intestine, and upregulated in colon, spleen and brown adipose tissue (BAT), compared to mice fed a control diet, supporting that SF4 is involved in the regulation of mammalian metabolism (see Fig. 4B). In one embodiment of the invention, expression of SF4 mRNA was also examined Non-Obese-Diabetic (NOD) mice. In those mice, the expression of SF4 is significantly downregulated in WAT, and upregulated in spleen, lung, muscle, liver, and testis, supporting that SF4 is involved in the regulation of mammalian metabolism (see Fig. 4C).

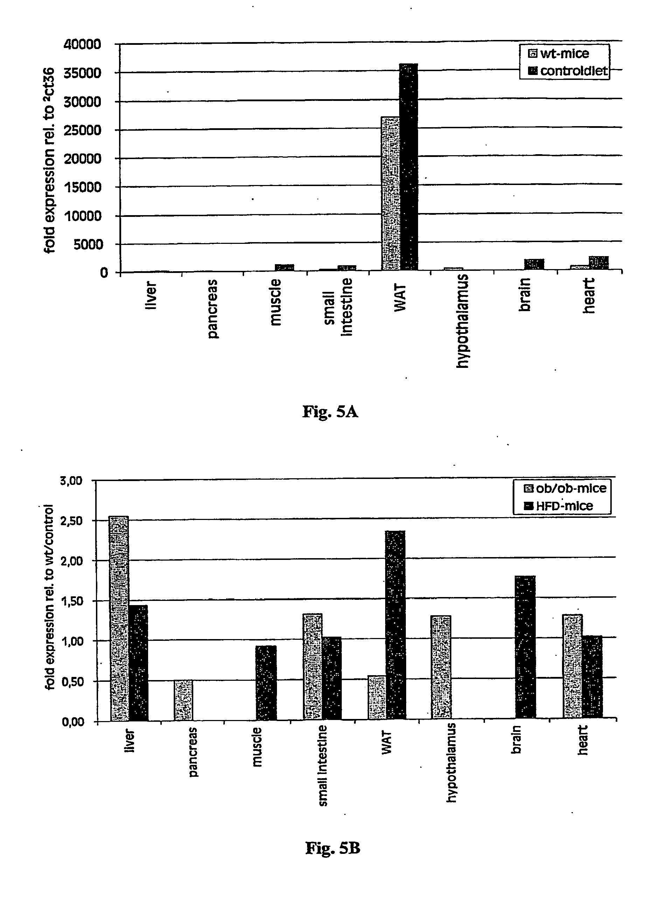

Taqman analysis revealed that SF5 is highly expressed in WAT. Furthermore SF5 is expressed on lower but still robust levels in the heart, brain, hypothalamus, small intestine, and muscle as depicted in Fig. 5A. Even though SF5 was expressed in the liver and pancreas of wild type mice on a low level, a clear up-regulation could be observed in the liver, and a down-regulation could be observed in the pancreas of ob/ob-mice. In wild type mice fed a high fat diet, the expression of SF5 was up-regulated in the WAT, when compared to mice fed a control diet (see Fig. 5B). The high expresssion level of SF5 in white adipose tissue of wild type and control mice as well as the regulation of gene expression in the mouse models for the metabolic syndrome as described above suggests that it might play a role in the regulation of energy homeostasis.

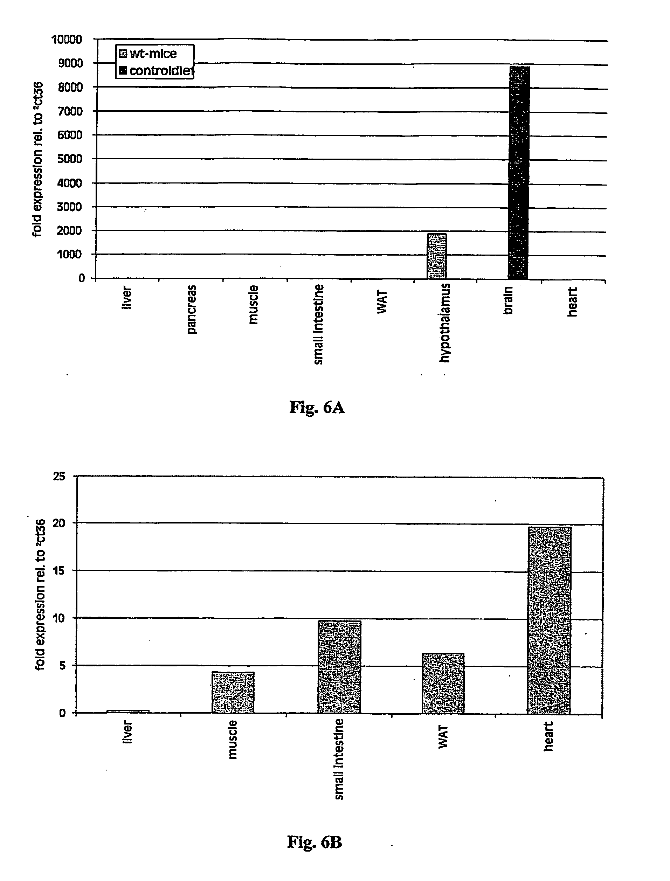

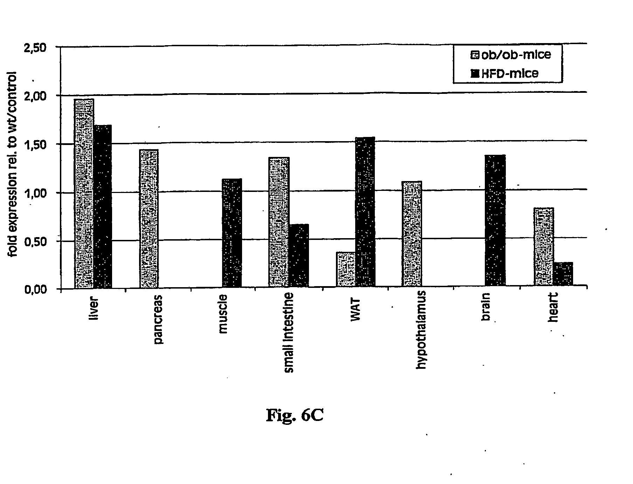

Taqman analysis revealed that SF6 is highly expressed in the hypothalamus and brain in wild type and control mice as shown in Fig. 6A. Furthermore SF6 is expressed on lower but still robust levels in the heart, small intestine, and WAT of control mice, as depicted in Fig. 6B. Even though SF6 was expressed in the heart and WAT of control mice on a low level, a down-regulation could be observed in the heart of mice fed a high fat diet and in the WAT of ob/ob mice (see Fig. 6C). The high expresssion level of SF6 in the hypothalamus and brain as well as the regulation of gene expression in mouse models for the metabolic syndrome as described above suggests that it might play a role in the regulation of energy homeostasis.

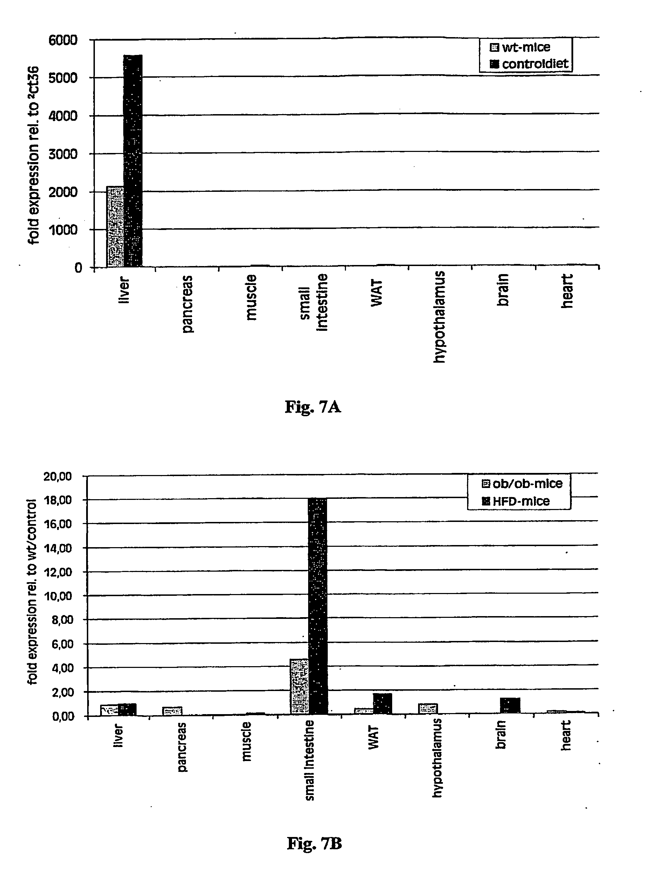

Taqman analysis revealed that SF7 is highly expressed in the liver of wild type and control diet mice. Furthermore SF7 is expressed on lower but still robust levels in the muscle, WAT, and heart as depicted in Fig. 7A. Even though SF7 was expressed in the WAT and heart of wild type mice on a low level, a down-regulation could be observed in the in the WAT and heart of ob/ob-mice. In wild type mice fed a high fat diet, the expression of SF7 was down-regulated in the muscle and heart, when compared to mice fed a control diet (see Fig. 7B). SF7 is expressed on a very low level in the small intestine of wild type mice but up-regulated to robust expression levels in the small intestine of ob/ob mice and HFD mice. The high expression level of SF7 in the liver of wild type and control mice as well as the regulation of gene expression in the mouse models for the metabolic syndrome as described above suggests that it might play a role in the regulation of energy homeostasis.

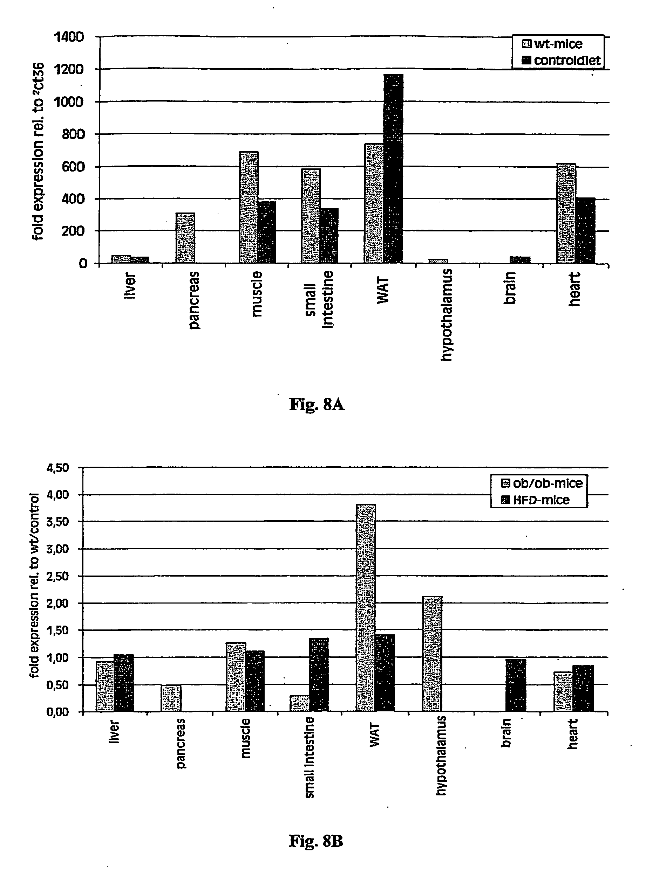

Taqman analysis revealed that SF8 is expressed in several mammalian tissues, showing highest level of expression in WAT, muscle, small intestine, heart, and pancreas. Furthermore SF8 is expressed on lower but still robust levels in the liver, hypothalamus, and brain as depicted in Fig. 8A. We found, for example, that the expression of SF8 is down-regulated in the pancreas and small intestine, and up-regulated in the WAT and hypothalamus of ob/ob mice compared to wild type mice (see Fig. 8B). In wild type mice fed a high fat diet, the expression of SF8 was not regulated. The expresssion of SF8 in metabolic active tissues of wild type and control mice as well as the regulation of gene expression in a mouse model for the metabolic syndrome as described above suggests that it might play a role in the regulation of energy homeostasis.

### Example 4: Analysis of the differential expression of transcripts of the SF3 and SF5 proteins in human tissues

RNA preparation from human primary adipose tissues was done as described in Example 3. The target preparation, hybridization, and scanning was performed as described in the manufactures manual (see Affymetrix Technical Manual, 2002, obtained from Affmetrix, Santa Clara, USA).

The expression analysis (using Affymetrix GeneChips) of the genes using primary human abdominal adipocycte differentiation clearly shows differential expression of the human SF3 and SF5 genes in adipocytes. Several independent experiments were done. In Fig. 9 and Fig. 10, the fluorescent intensity is shown on the Y-axis, and the X-axis represents the time axis. "d 0" refers to day 0 (start of the experiment), "d12" refers to day 12 of adipocyte differentiation.

The experiments show that the SF3 transcript is most abundant at day 12 compared to day 0 during differentiation (see Fig. 9). The experiments also show that the SF5 transcript is most abundant at day 0 compared to day 12 during differentiation (see Fig. 10A and Fig. 10B).

Thus, the SF3 protein has to be increased in order for the preadipocyctes to differentiate into mature adipocyctes, and the SF5 protein hse to be decreased in order for the preadipocyctes to differentiate into mature adipocyctes. The SF3 protein in preadipocyctes has the potential to enhance adipose differentiation, and the SF5 protein in preadipocyctes has the potential to inhibit adipose differentiation. Therefore, the SF3 and SF5 proteins might play an essential role in the regulation of human metabolism, in particular in the regulation of adipogenesis and thus it might be an essential role in pancreatic diseases (e.g. diabetes), obesity, and/or metabolic syndrome.

### Example 5: In situ hybridisations

SF1 is a protein which was isolated in a screen for secreted proteins expressed in the developing mouse pancreas during embryogenesis. The isolation of the corresponding cDNA from an embryonic mouse pancreas library demonstrates that the gene is expressed in this tissue at this time. To gain more information about the expression of this gene, in situ hybridization was performed on sections of embryonic mouse pancreas (day E17.5) according to standard protocols as known to those skilled in the art and as described previously (for example, Pelton, R.W. et al., (1990) Development 110: 609-620; Belo, J.A. et al., (1997) Mech. Dev. 68: 45-57), combined with antibody stainings for insulin (beta cell marker) or DBA lectin (duct cell marker).

SF1 showed widespread expression in non-beta cell and non-duct cells. The distribution and shape of the expressing cells as well as co-staining with the exocrine marker amylase indicate that SF1 is expressed predominantly in exocrine cells in the developing pancreas.

The formation of beta cells during embryogenesis or during regenerative processes after birth is a tightly controlled process. The signalling molecules involved in the control of this process are not known completely. It has been shown that the formation of novel beta cells is regulated by signals produced by other cell types in the pancreas, for example endothelial cells (Lammert E, et al., (2001) Science 294: 564-567). Thus, SF1 is a candidate regulator of the formation of pancreatic endocrine and other pancreatic cell types. It is noteworthy that despite their homology to chitinases, homologs of SF1 act as growth factors in Drosophila (Kawamura et al., supra).

The subject-matter of the following items is also disclosed herein.
1. A pharmaceutical composition comprising a SF1, SF2, SF3, SF4, SF5, SF6, SF7, or SF8 protein and/or a functional fragment thereof, a nucleic acid molecule encoding a SF1, SF2, SF3, SF4, SF5, SF6, SF7, or SF8 protein and/or a functional fragment thereof and/or an effector/modulator of said nucleic acid molecule and/or said protein or protein fragment.
2. The composition of item 1, wherein the composition contains pharmaceutically acceptable carriers, diluents, and/or additives.
3. The composition of item 1 or 2, wherein the nucleic acid molecule is a mammalian SF1, SF2, SF3, SF4, SF5, SF6, SF7, or SF8 nucleic acid, particularly encoding a human SF1, SF2, SF3, SF4, SF5, SF6, SF7, or SF8 polypeptide and/or a nucleic molecule, which is complementary thereto or a fragment thereof or a variant thereof.
4. The composition of any one of items 1 to 3, wherein said nucleic acid molecule is selected from the group consisting of
   (a) a nucleic acid molecule encoding a polypeptide as shown in Table 1, or an isoform, fragment or variant of the polypeptide as shown in Table 1;
   (b) a nucleic acid molecule which comprises or is the nucleic acid molecule as shown in Table 1;
   (c) a nucleic acid molecule being degenerate with as a result of the genetic code to the nucleic acid sequences as defined in (a) or (b),
   (d) a nucleic acid molecule that hybridizes at 50°C in a solution containing 1 x SSC and 0.1% SDS to a nucleic acid molecule as defined in claim 3 or as defined in (a) to (c) and/or a nucleic acid molecule which is complementary thereto;
   (e) a nucleic acid molecule that encodes a polypeptide which is at least 85%, preferably at least 90%, more preferably at least 95%, more preferably at least 98% and up to 99,6% identical to the human SF1, SF2, SF3, SF4, SF5, SF6, SF7, or SF8, as defined in claim 3 or to a polypeptide as defined in (a);
   (f) a nucleic acid molecule that differs from the nucleic acid molecule of (a) to (e) by mutation and wherein said mutation causes an alteration, deletion, duplication or premature stop in the encoded polypeptide
5. The composition of any one of items 1-4, wherein the nucleic acid molecule is a DNA molecule, particularly a cDNA or a genomic DNA.
6. The composition of any one of items 1-5, wherein said nucleic acid encodes a polypeptide contributing to regulating the metabolism, in particular human metabolism.
7. The composition of any one of items 1-6, wherein said nucleic acid molecule is a recombinant nucleic acid molecule.
8. The composition of any one of items 1-7, wherein the nucleic acid molecule is a vector, particularly an expression vector.
9. The composition of any one of items 1-6, wherein the polypeptide is a recombinant polypeptide.
10. The composition of item 9, wherein said recombinant polypeptide is a fusion polypeptide.
11. The composition of any one of items 1-8, wherein said nucleic acid molecule is selected from hybridization probes, primers and anti-sense oligonucleotides.
12. The composition of any one of items 1-11 which is a diagnostic composition.
13. The composition of any one of items 1-11 which is a therapeutic composition.
14. The composition of any one of items 1-13 for the manufacture of an agent for detecting and/or verifying, for the treatment, alleviation and/or prevention of pancreatic diseases (e.g. diabetes such as insulin dependent diabetes mellitus, non insulin dependent diabetes mellitus or latent autoimmune diabetes in adults), obesity, metabolic syndrome and/or other metabolic diseases or dysfunctions.
15. The composition of any one of items 1-14 for the manufacture of an agent for the modulation of pancreatic development.
16. The composition of any one of items 1-15 for the manufacture of an agent for the regeneration of pancreatic tissues or cells, particularly pancreatic cells, more particularly beta cells, or exocrine cells.
17. The composition of any one of items 1-16 for application in vivo.
18. The composition of any one of items 1-16 for application in vitro.
19. The composition of any one of items 1-18 in combination with at least one further pharmaceutical agent.
20. The composition of item 19 wherein the at least one further pharmaceutical agent is an agent suitable for the prevention and/or treatment of pancreatic diseases and/or obesity and/or metabolic syndrome.
21. The composition of item 19 or 20 wherein the at least one further pharmaceutical agent is an immunosuppressive agent.
22. Use of a SF1, SF2, SF3, SF4, SF5, SF6, SF7, or SF8 nucleic acid molecule or a polypeptide encoded thereby or a fragment or a variant of said nucleic acid molecule or said polypeptide and/or an effector/modulator of said nucleic acid or polypeptide for the manufacture of a medicament for the treatment of pancreatic diseases (e.g. diabetes such as insulin dependent diabetes mellitus, non insulin dependent diabetes mellitus or latent autoimmune diabetes in adults), obesity, metabolic syndrome and/or other metabolic diseases or dysfunctions for controlling the function of a gene and/or a gene product which is influenced and/or modified by a SF1, SF2, SF3, SF4, SF5, SF6, SF7, or SF8 polypeptide.
23. The use of item 22 in combination with at least one further pharmaceutical agent.
24. Use of the SF1, SF2, SF3, SF4, SF5, SF6, SF7, or SF8 nucleic acid molecule or use of a polypeptide encoded thereby, or use of a fragment or a variant of said nucleic acid molecule or said polypeptide, or use of an effector/modulator of said nucleic acid molecule or said polypeptide for identifying substances capable of interacting with a SF1, SF2, SF3, SF4, SF5, SF6, SF7, or SF8 polypeptide in vitro and/or in vivo.
25. A non-human transgenic animal exhibiting a modified expression of a SF1, SF2, SF3, SF4, SF5, SF6, SF7, or SF8 polypeptide.
26. The animal of item 25, wherein the expression of the SF1, SF2, SF3, SF4, SF5, SF6, SF7, or SF8 polypeptide is increased and/or reduced.
27. A recombinant host cell exhibiting a modified expression of a SF1, SF2, SF3, SF4, SF5, SF6, SF7, or SF8 polypeptide, or a recombinant host cell which comprises a nucleic acid molecule as defined in any one of items 1 to 7.
28. The cell of item 27 which is a human cell.
29. A method of identifying a (poly)peptide involved in the regulation of energy homeostasis and/or metabolism in a mammal comprising the steps of
   (a) contacting a collection of (poly)peptides with a SF1, SF2, SF3, SF4, SF5, SF6, SF7, or SF8 homologous polypeptide or a fragment thereof under conditions that allow binding of said (poly)peptides;
   (b) removing (poly)peptides which do not bind and
   (c) identifying (poly)peptides that bind to said SF1, SF2, SF3, SF4, SF5, SF6, SF7, or SF8 homologous polypeptide.
30. A method of screening for an agent which effects/modulates the interaction of a SF1, SF2, SF3, SF4, SF5, SF6, SF7, or SF8 polypeptide with a binding target comprising the steps of
   (a) incubating a mixture comprising
   (aa) a SF1, SF2, SF3, SF4, SF5, SF6, SF7, or SF8 polypeptide or a fragment thereof;
   (ab) a binding target/agent of said SF1, SF2, SF3, SF4, SF5, SF6, SF7, or SF8 polypeptide or fragment thereof; and
   (ac) a candidate agent under conditions whereby said polypeptide or fragment thereof specifically binds to said binding target at a reference affinity;
   (b) detecting the binding affinity of said SF1, SF2, SF3, SF4, SF5, SF6, SF7, or SF8 polypeptide or fragment thereof to said binding target to determine an affinity for the agent; and
   (c) determining a difference between affinity for the agent and reference affinity.
31. A method for screening for an agent, which effects/modulates the activity of a SF1, SF2, SF3, SF4, SF5, SF6, SF7, or SF8 polypeptide, comprising the steps of
   (a) incubating a mixture comprising
   (aa) a SF1, SF2, SF3, SF4, SF5, SF6, SF7, or SF8 polypeptide or a fragment thereof; and
   (ab) a candidate agent under conditions whereby said SF1, SF2, SF3, SF4, SF5, SF6, SF7, or SF8 polypeptide or fragment thereof exhibits a reference activity,
   (b) detecting the activity of said SF1, SF2, SF3, SF4, SF5, SF6, SF7, or SF8 polypeptide or fragment thereof to determine an activity for the agent; and
   (c) determining a difference between activity for the agent and reference activity.
32. A method of producing a composition comprising the (poly)peptide identified by the method of item 29 or the agent identified by the method of item 30 or 31 with a pharmaceutically acceptable carrier and/or diluent.
33.The method of item 32 wherein said composition is a pharmaceutical composition for preventing, alleviating or treating of diseases and disorders, including pancreatic diseases (e.g. diabetes), obesity, and/or metabolic syndrome.
34. Use of a (poly)peptide as identified by the method of item 29 or of an agent as identified by the method of item 30 or 31 for the preparation of a pharmaceutical composition (i) for the treatment, alleviation and/or prevention of pancreatic diseases (e.g. diabetes), obesity, and/or metabolic syndrome, (ii) for the modulation of pancreatic development and/or (iii) for the regeneration of pancreatic cells or tissues.
35. Use of a nucleic acid molecule as defined in any one of items 1 to 7 or 11 for the preparation of a medicament (i) for the treatment, alleviation and/or prevention of diseases or dysfunctions, including pancreatic diseases (e.g. diabetes), obesity, and/or metabolic syndrome, (ii) for the modulation of pancreatic development and/or (iii) for the regeneration of pancreatic cells or tissues.
36. Use of a polypeptide as defined in any one of items 1 to 6, 9 or 10 for the preparation of a medicament (i) for the treatment, alleviation and/or prevention of pancreatic diseases (e.g. diabetes), obesity, and/or metabolic syndrome, (ii) for the modulation of pancreatic development and/or (iii) for the regeneration of pancreatic cells or tissues.
37.Use of a vector as defined in item 8 for the preparation of a medicament (i) for the treatment, alleviation and/or prevention of pancreatic diseases (e.g. diabetes), obesity, and/or metabolic syndrome, (ii) for the modulation of pancreatic development and/or (iii) for the regeneration of pancreatic cells or tissues.
38. Use of a host cell as defined in item 27 or 28 for the preparation of a medicament (i) for the treatment, alleviation and/or prevention of pancreatic diseases (e.g. diabetes), obesity, and/or metabolic syndrome, (ii) for the modulation of pancreatic development and/or (iii) for the regeneration of pancreatic cells or tissues.
39. The use of any one of items 34-38 wherein the medicament comprises at least one further pharmaceutical agent.
40. Use of a SF1, SF2, SF3, SF4, SF5, SF6, SF7, or SF8 nucleic acid molecule or of a fragment thereof for the production of a non-human transgenic animal which over- or under-expresses the SF1, SF2, SF3, SF4, SF5, SF6, SF7, or SF8 gene product.
41. Kit comprising at least one of
   (a) a SF1, SF2, SF3, SF4, SF5, SF6, SF7, or SF8 nucleic acid molecule or a functional fragment or an isoform thereof;
   (b) a SF1, SF2, SF3, SF4, SF5, SF6, SF7, or SF8 amino acid molecule or a functional fragment or an isoform thereof;
   (c) a vector comprising the nucleic acid of (a);
   (d) a host cell comprising the nucleic acid of (a) or the vector of (b);
   (e) a polypeptide encoded by the nucleic acid of (a), expressed by the vector of (c) or the host cell of (a);
   (f) a fusion polypeptide encoded by the nucleic acid of (a);
   (g) an antibody, an aptamer or another effector/modulator against the nucleic acid of (a) or the polypeptide of (b), (e), or (f) and/or
   (h) an anti-sense oligonucleotide of the nucleic acid of (a).

## Claims

1. A pharmaceutical composition for use in the treatment of diabetes and/or obesity, comprising an SF6 protein and/or a nucleic acid molecule encoding an SF6 protein and/or an effector/modulator of said nucleic acid molecule and/or said protein, wherein the nucleic acid molecule is a mammalian SF6 nucleic acid, particularly encoding a human SF6 polypeptide and/or a nucleic acid molecule, which is complementary thereto or a fragment thereof or constructs expressing anti-sense and/or dominant-negative mutated molecules, and wherein the composition optionally contains pharmaceutically acceptable carriers, diluents, and/or additives, wherein the effector/modulator is selected from antibodies which are specific for a SF6 protein, as well as antisense molecules, aptamers, RNAi molecules and/or ribozymes recognising said SF6 protein or nucleic acid molecule encoding an SF6 protein.

2. The composition for use according to claim 1, wherein said nucleic acid molecule is selected from the group consisting of
(a) a nucleic acid molecule encoding a polypeptide having GenBank Accession No. NP_058023 or NP_006150, or an isoform, or fragment of the polypeptide having GenBank Accession No. NP_058023 or NP_006150;
(b) a nucleic acid molecule which comprises or is the nucleic acid molecule having GenBank Accession No. NM_016743 or NM_006159;
(c) a nucleic acid molecule being degenerate with as a result of the genetic code to the nucleic acid sequences as defined in (a) or (b),
(d) a nucleic acid molecule that hybridizes at 50°C in a solution containing 1 x SSC and 0.1% SDS to a nucleic acid molecule as defined in claim 1 or as defined in (a) to (c) and/or a nucleic acid molecule which is complementary thereto;
(e) a nucleic acid molecule that encodes a polypeptide which is at least 85%, preferably at least 90%, more preferably at least 95%, more preferably at least 98% and up to 99,6% identical to the human SF6 as defined in claim 1 or to a polypeptide as defined in (a).

3. The composition for use according to any one of claims 1-2 in combination with at least one further pharmaceutical agent, wherein the at least one further pharmaceutical agent is particularly an agent suitable for the prevention and/or treatment of pancreatic diseases and/or obesity.

4. SF6 nucleic acid molecule or a polypeptide encoded thereby or a fragment of said nucleic acid molecule or said polypeptide and/or an effector/modulator of said nucleic or polypeptide for use in the treatment of diabetes, and/or obesity, wherein the effector/modulator is selected from antibodies, which are specific for a SF6 protein, as well as antisense molecules, aptamers, RNAi molecules and/or ribozymes recognising said SF6 protein or nucleic acid molecule encoding an SF6 protein.

5. A method of identifying a (poly)peptide involved in the regulation of energy homeostasis and/or metabolism in a mammal comprising the steps of
(a) contacting a collection of (poly)peptides with an SF6 homologous polypeptide or a fragment thereof under conditions that allow binding of said (poly)peptides;
(b) removing (poly)peptides which do not bind and
(c) identifying (poly)peptides that bind to said SF6 homologous polypeptide, wherein a SF6 homologous polypeptide or fragment thereof is encoded by a nucleic acid as defined in
(i) a nucleic acid molecule encoding a polypeptide having GenBank Accession No. NP_058023 or NP_006150, or an isoform, or fragment of the polypeptide having GenBank Accession No. NP_058023 or NP_006150;
(ii) a nucleic acid molecule which comprises or is the nucleic acid molecule having GenBank Accession No. NM_016743 or NM_006159;
(iii) a nucleic acid molecule being degenerate with as a result of the genetic code to the nucleic acid sequences as defined in (i) or (ii),
(iv) a nucleic acid molecule that hybridizes at 50°C in a solution containing 1 x SSC and 0.1% SDS to a nucleic acid molecule as defined in (i) to (iii) and/or a nucleic acid molecule which is complementary thereto;
(v) a nucleic acid molecule that encodes a polypeptide which is at least 85%, preferably at least 90%, more preferably at least 95%, more preferably at least 98% and up to 99,6% identical to the human SF6 as defined in claim 1 or to a polypeptide as defined in (i).

6. A method of screening for an agent which effects/modulates the interaction of an SF6 polypeptide with a binding target comprising the steps of
(a) incubating a mixture comprising
(aa) an SF6 polypeptide or a fragment thereof;
(ab) a binding target/agent of said SF6 polypeptide or fragment thereof; and
(ac) a candidate agent under conditions whereby said polypeptide or fragment thereof specifically binds to said binding target at a reference affinity;
(b) detecting the binding affinity of said SF6 polypeptide or fragment thereof to said binding target to determine an affinity for the agent; and
(c) determining a difference between affinity for the agent and reference affinity.

7. A method for screening for an agent, which effects/modulates the activity of an SF6 polypeptide, comprising the steps of
(a) incubating a mixture comprising
(aa) an SF6 polypeptide or a fragment thereof; and
(ab) a candidate agent
under conditions whereby said SF6 polypeptide or fragment thereof exhibits a reference activity,
(b) detecting the activity of said SF6 polypeptide or fragment thereof to determine an activity for the agent; and
(c) determining a difference between activity for the agent and reference activity.

8. Nucleic acid molecule
which is a
mammalian SF6 nucleic acid, particularly encoding a human SF6 polypeptide, and/or a nucleic acid which is complementary thereto, or a fragment thereof or constructs expressing anti-sense and/or dominant-negative mutated molecules,
a SF6 polypeptide and/or
a recombinant host cell exhibiting a modified expression of an SF6 polypeptide, or a recombinant host cell comprising a nucleic acid molecule, which is a mammalian SF6 nucleic acid which is complementary thereto, or a fragment thereof or constructs expressing anti-sense and/or dominant-negative mutated molecules, wherein the host cell is particularly a human cell,
for use in the treatment, alleviation and/or prevention of diabetes, and/or obesity.

9. Kit comprising at least one of
(a) an SF6 nucleic acid molecule or an isoform thereof;
(b) an SF6 amino acid molecule or an isoform thereof;
(c) a vector comprising the nucleic acid of (a);
(d) a host cell comprising the nucleic acid of (a) or the vector of (c);
(e) a polypeptide encoded by the nucleic acid of (a), expressed by the vector of (c) or the host cell of (d);
(f) a fusion polypeptide encoded by the nucleic acid of (a);
(g) an antibody, an aptamer or another effector/modulator against the nucleic acid of (a) or the polypeptide of (b), (e), or (f) and /or
(h) an anti-sense oligonucleotide of the nucleic acid of (a).

## Patentansprüche

1. Pharmazeutische Zusammensetzung zur Verwendung in der Behandlung von Diabetes und/oder Fettleibigkeit, umfassend ein SF6-Protein und/oder ein Nukleinsäuremolekül, das für ein SF6-Protein kodiert und/oder einen Effektor/Modulator des Nukleinsäuremoleküls und/oder des Proteins, wobei das Nukleinsäuremolekül eine SF6-Nukleinsäure aus Säugern ist, die insbesondere für ein humanes SF6-Polypeptid kodiert, und/oder ein Nukleinsäuremolekül, das komplementär dazu ist, oder ein Fragment davon oder Konstrukte, die anti-sense und/oder dominant-negativ mutierte Moleküle exprimieren, und wobei die Zusammensetzung gegebenenfalls pharmazeutisch akzeptable Träger, Verdünnungsmittel, und/oder Zusatzstoffe enthält, wobei der Effektor/Modulator ausgewählt ist aus Antikörpern, die spezifisch für ein SF6-Protein sind, ebenso wie Antisense-Molekülen, Aptameren, RNAi-Molekülen und/oder Ribozymen, die das SF6-Protein oder das Nukleinsäuremolekül, das für ein SF6-Protein kodiert, erkennen.

2. Zusammensetzung zur Verwendung nach Anspruch 1, wobei das Nukleinsäuremolekül ausgewählt ist aus der Gruppe bestehend aus
(a) einem Nukleinsäuremolekül, das für ein Polypeptid kodiert, das die GenBank Zugangsnr. NP_058023 oder NP_006150 aufweist, oder eine Isoform oder ein Fragment des Polypeptids, das die GenBank Zugangsnr. NP_058023 oder NP_006150 aufweist;
(b) einem Nukleinsäuremolekül, das das Nukleinsäuremolekül, das die GenBank Zugangsnr. NM_016743 oder NM_006159 aufweist, umfasst oder ist;
(c) einem Nukleinsäuremolekül, das aufgrund des genetischen Codes degeneriert zu den Nukleinsäuresequenzen wie in (a) oder (b) definiert ist;
(d) einem Nukleinsäüremolekül, das bei 50°C in einer Lösung, die 1 x SSC und 0,1% SDS enthält, an ein Nukleinsäuremolekül wie in Anspruch 1 definiert oder wie in (a) bis (c) definiert, hybridisiert, und/oder einem Nukleinsäuremolekül, das dazu komplementär ist;
(e) einem Nukleinsäuremolekül, das für ein Polypeptid kodiert, das mindestens 85%, bevorzugt mindestens 90%, stärker bevorzugt mindestens 95%, stärker bevorzugt mindestens 98% und bis zu 99,6% identisch zum humanen SF6-Protein wie in Anspruch 1 definiert oder zu einem Polypeptid wie in (a) definiert ist.

3. Zusammensetzung zur Verwendung nach einem der Ansprüche 1-2 in Kombination mit mindestens einem weiteren pharmazeutischen Wirkstoff, wobei der mindestens eine weitere pharmazeutische Wirkstoff insbesondere ein Wirkstoff ist, der geeignet ist zur Prävention und/oder Behandlung von Erkrankungen der Bauchspeicheldrüse und/oder Fettleibigkeit.

4. SF6-Nukleinsäuremolekül oder ein davon kodiertes Polypeptid oder ein Fragment dieses Nukleinsäuremoleküls oder Polypeptids, und/oder ein Effektor/Modulator der Nukleinsäure oder des Polypeptids zur Verwendung in der Behandlung von Diabetes und/oder Fettleibigkeit, wobei der Effektor/Modulator ausgewählt ist aus Antikörpern, die spezifisch für ein SF6-Protein sind, ebenso wie Antisense-Molekülen, Aptameren, RNAi-Molekülen und/oder Ribozymen, die das SF6-Protein oder das Nukleinsäuremolekül, das für ein SF6-Protein kodiert, erkennen.

5. Verfahren zur Identifizierung eines (Poly)Peptids, das an der Regulation von Energiehomöostase und/oder -metabolismus in einem Säuger beteiligt ist, umfassend die Schritte
(a) Inkontaktbringen einer Sammlung von (Poly)Peptiden mit einem SF6-homologen Polypeptid oder einem Fragment davon unter Bedingungen, die eine Bindung dieser (Poly)Peptide erlauben;
(b) Entfernen von (Poly)Peptiden, die nicht binden, und
(c) Identifizieren von (Poly)Peptiden, die an das SF6-homologe Polypeptid binden, wobei ein SF6-homologes Polypeptid oder Fragment davon von einer Nukleinsäure kodiert wird wie definiert in
(i) einem Nukleinsäuremolekül, das für ein Polypeptid kodiert, das die GenBank Zugangsnr. NP_058023 oder NP_006150 aufweist, oder eine Isoform oder ein Fragment des Polypeptids, das die GenBank Zugangsnr. NP_058023 oder NP_006150 aufweist;
(ii) einem Nukleinsäuremolekül, das das Nukleinsäuremolekül, das die GenBank Zugangsnr. NM_016743 oder NM_006159 aufweist, umfasst oder ist;
(iii) einem Nukleinsäuremolekül, das aufgrund des genetischen Codes degeneriert zu den Nukleinsäuresequenzen wie in (i) oder (ii) definiert ist;
(iv) einem Nukleinsäuremolekül, das bei 50°C in einer Lösung, die 1 x SSC und 0,1 % SDS enthält, an ein Nukleinsäuremolekül wie in (i) bis (iii) definiert, hybridisiert, und/oder einem Nukleinsäuremolekül, das dazu komplementär ist;
(v) einem Nukleinsäuremolekül, das für ein Polypeptid kodiert, das mindestens 85%, bevorzugt mindestens 90%, stärker bevorzugt mindestens 95%, stärker bevorzugt mindestens 98% und bis zu 99,6% identisch zum humanen SF6-Protein wie in Anspruch 1 definiert oder zu einem Polypeptid wie in (i) definiert ist.

6. Verfahren zum Screening für einen Wirkstoff, das die Interaktion eines SF6-Polypeptids mit einem Bindungs-Target beeinflusst/moduliert, umfassend die Schritte
(a) Inkubieren einer Mischung umfassend
(aa) ein SF6-Polypeptid oder ein Fragment davon;
(ab) ein(en) Bindungs-Target/-wirkstoff des SF6-Polypeptids oder Fragments davon; und
(ac) einen Wirkstoffkandidaten, unter Bedingungen, bei denen das Polypeptid oder Fragment davon spezifisch an das Bindungs-Target bei einer Referenzaffinität bindet;
(b) Detektieren der Bindungsaffinität des SF6-Polypeptids oder Fragments davon an das Bindungs-Target, um eine Affinität für den Wirkstoff zu bestimmen; und
(c) Bestimmen eines Unterschieds zwischen Affinität für den Wirkstoff und Referenzaffinität.

7. Verfahren zum Screening für einen Wirkstoff, das die Aktivität eines SF6-Polypeptids beeinflusst/moduliert, umfassend die Schritte
(a) Inkubieren einer Mischung umfassend
(aa) ein SF6-Polypeptid oder ein Fragment davon; und
(ab) einen Wirkstoffkandidaten,
unter Bedingungen, bei denen das SF6-Polypeptid oder Fragment davon eine Referenzaktivität zeigt;
(b) Detektieren der Aktivität des SF6-Polypeptids oder Fragments davon, um eine Aktivität für den Wirkstoff zu bestimmen; und
(c) Bestimmen eines Unterschieds zwischen Aktivität für den Wirkstoff und Referenzaktivität.

8. Nukleinsäuremolekül,
das eine SF6-Nukleinsäure aus Säugern ist, die insbesondere für ein humanes SF6-Polypeptid kodiert, und/oder eine Nukleinsäure, die komplementär dazu ist, oder ein Fragment davon oder Konstrukte, die anti-sense und/oder dominant-negativ mutierte Moleküle exprimieren, ein SF6-Polypeptid und/oder
eine rekombinante Wirtszelle, die eine modifizierte Expression eines SF6-Polypeptids zeigt, oder eine rekombinante Wirtszelle, die eine Nukleinsäure umfasst, die eine SF6-Nukleinsäure aus Säugern ist,
oder ein Fragment davon oder Konstrukte, die anti-sense und/oder dominant-negativ mutierte Moleküle exprimieren, wobei die Wirtszelle insbesondere eine humane Zelle ist,
zur Verwendung in der Behandlung, Linderung, und/oder Prävention von Diabetes und/oder Fettleibigkeit.

9. Kit umfassend mindestens eines aus
(a) einem SF6-Nukleinsäuremolekül oder einer Isoform davon;
(b) einem SF6-Aminosäuremolekül oder einer Isoform davon;
(c) einem Vektor umfassend die Nukleinsäure aus (a)
(d) einer Wirtszelle umfassend die Nukleinsäure aus (a) oder den Vektor aus (c);
(e) einem Polypeptid, das von der Nukleinsäure aus (a) kodiert wird, exprimiert vom Vektor aus (c) oder der Wirtszelle aus (d);
(f) einem Fusionspolypeptid, das von der Nukleinsäure aus (a) kodiert wird;
(g) einem Antikörper, einem Aptamer oder einem anderen Effektor/Modulator gegen die Nukleinsäure aus (a) oder das Polypeptid aus (b), (e) oder (f), und/oder
(h) einem anti-sense-Oligonukleotid der Nukleinsäure aus (a).

## Revendications

1. Composition pharmaceutique pour une utilisation dans le traitement du diabète et/ou de l'obésité, comprenant une protéine SF6 et/ou une molécule d'acide nucléique codant pour une protéine SF6 et/ou un effecteur/modulateur de ladite molécule d'acide nucléique et/ou ladite protéine, dans laquelle la molécule d'acide nucléique est un acide nucléique SF6 de mammifère, particulièrement codant pour le polypeptide SF6 humain et/ou une molécule d'acide nucléique, qui en est complémentaire ou un fragment de celle-ci, ou des construits exprimant des molécules anti-sens et/ou mutées à dominant négatif, et dans laquelle la composition contient optionnellement des vecteurs, des diluants, et/ou des additifs, dans laquelle l'effecteur/modulateur est sélectionné parmi les anticorps qui sont spécifiques à la protéine SF6 ainsi que des molécules anti-sens, des aptamères, des molécules d'ARNi et/ou des ribozymes reconnaissant ladite protéine SF6 ou ladite molécule d'acide nucléique codant pour la protéine SF6.

2. Composition pour une utilisation selon la revendication 1, dans laquelle ladite molécule d'acide nucléique est sélectionnée parmi le groupe consistant en
(a) une molécule d'acide nucléique codant pour un polypeptide ayant un numéro de dépôt GenBank NP_058023 ou NP_006150, ou un isoforme, ou un fragment de polypeptide ayant un numéro de dépôt GenBank NP_058023 ou NP_006150;
(b) une molécule d'acide nucléique qui comprend ou est une molécule d'acide nucléique ayant un numéro de dépôt Genbank NM_016743 ou NM_006159;
(c) une molécule d'acide nucléique étant dégénérée avec, en tant que résultat du code génétique, des séquences d'acide nucléique telles que définies en (a) ou (b),
(d) une molécule d'acide nucléique qui s'hybride à 50°C dans une solution contenant 1 x SSC et 0,1% SDS en une molécule d'acide nucléique telle que définie dans la revendication 1 ou telle que définie en (a) à (c) et/ou une molécule d'acide nucléique qui en est complémentaire;
(e) une molécule d'acide nucléique qui code pour un polypeptide qui est au moins 85%, de préférence au moins 90%, encore de préférence au moins 95%, encore de préférence au moins 98% et jusqu'à 99,6% identique à la SF6 humaine tel que définie dans la revendication 1 ou à un polypeptide tel que défini dans (a).

3. Composition pour une utilisation selon une quelconque des revendications 1-2 en combinaison avec au moins un agent pharmaceutique supplémentaire, dans laquelle l'agent pharmaceutique au moins supplémentaire est en particulier un agent approprié pour la prévention et/ou le traitement de maladies pancréatiques et/ou de l'obésité.

4. Molécule d'acide nucléique SF6 ou polypeptide la codant ou fragment de ladite molécule d'acide nucléique ou dudit polypeptide et/ou un effecteur/modulateur de ladite molécule d'acide nucléique ou polypeptide pour une utilisation dans le traitement du diabète, et/ou de l'obésité, dans laquelle l'effecteur/modulateur est sélectionné parmi des anticorps, qui sont spécifiques pour une protéine SF6 ainsi que des molécules anti-sens, des aptamères, des molécules d'ARNi et/ou des ribozymes reconnaissant ladite protéine SF6 ou ladite molécule d'acide nucléique codant pour la protéine SF6.

5. Méthode d'identification d'un (poly)peptide impliqué dans la régulation de l'homéostasie énergétique et/ou le métabolisme chez un mammifère comprenant les étapes de
(a) contacter une collection de (poly)peptides avec un polypeptide homologue SF6 ou un fragment de celui-ci dans des conditions qui permettent la liaison auxdits (poly)peptides;
(b) retirer les (poly)peptides qui ne se lient pas et
(c) identifier les (poly)peptides qui se lient audit polypeptide homologue SF6, dans lequel un polypeptide homologue SF6 ou un fragment de celui-ci est codé par un acide nucléique tel que défini dans
(i) une molécule d'acide nucléique codant pour un polypeptide ayant un numéro de dépôt GenBank NP_058023 ou NP_006150, ou un isoforme, ou un fragment du polypeptide ayant un numéro de dépôt GenBank NP_058023 ou NP_006150;
(ii) une molécule d'acide nucléique qui comprend ou qui est une molécule d'acide nucléique ayant un numéro de dépôt GenBank NM_016743 ou NM_006159;
(iii) une molécule d'acide nucléique étant dégénérée avec, en tant que résultat du code génétique, des séquences d'acide nucléique telles que définies dans (i) ou (ii),
(iv) une molécule d'acide nucléique qui s'hybride à 50°C dans une solution contenant 1 x SSC et 0,1% SDS en une molécule d'acide nucléique telle que définie dans la revendication 1 ou telle que définie en (i) à (iii) et/ou une molécule d'acide nucléique qui en est complémentaire;
(v) une molécule d'acide nucléique qui code pour un polypeptide qui est au moins 85%, de préférence au moins 90%, encore de préférence au moins 95%, encore de préférence au moins 98% et jusqu'à 99,6% identique à la SF6 humaine telle que définie dans la revendication 1 ou à un polypeptide tel que défini dans (i).

6. Méthode de criblage d'un agent qui effectue/module l'intéraction d'un polypeptide SF6 avec une cible de liaison comprenant les étapes
(a) incuber le mélange comprenant
(aa) un polypeptide SF6 ou un fragment de celui-ci;
(ab) une cible / un agent de liaison dudit polypeptide SF6 ou un fragment de celui-ci; et
(ac) un agent candidat dans des conditions dans lesquelles ledit polypeptide ou fragment de celui-ci se lie de façon spécifique à ladite cible de liaison à une affinité de référence;
(b) détecter l'affinité de liaison dudit polypeptide SF6 ou fragment de celui-ci à ladite cible pour déterminer une affinité pour l'agent; et
(c) déterminer une différence entre l'affinité pour l'agent et l'affinité de référence.

7. Méthode de criblage d'un agent, qui effectue/module l'activité d'un polypeptide SF6, comprenant les étapes de
(a) incuber le mélange comprenant
(aa) un polypeptide SF6 ou un fragment de celui-ci;
(ab) un agent candidat
dans des conditions dans lesquelles ledit polypeptide SF6 ou le fragment de celui-ci montre une activité de référence,
(b) détecter l'activité dudit polypeptide SF6 ou du fragment de celui-ci pour déterminer une activité pour cet agent; et
(c) déterminer une différence entre l'activité de l'agent et l'activité de référence.

8. Molécule d'acide nucléique qui est
un acide nucléique SF6 de mammifère, particulèrement codant pour un polypeptide humain SF6, et/ou un acide nucléique qui en est complémentaire, ou un fragment de celui-ci ou des construits exprimant des molécules anti-sens et/ou mutées à dominant négatif,
un polypeptide SF6 et/ou
une cellule hôte recombinante exprimant une expression modifiée d'un polypeptide SF6, ou une cellule hôte recombinante comprenant une molécule d'acide nucléique, qui est une molécule d'acide nucléique SF6 de mammifère qui en est complémentaire, ou un fragment ou des construits exprimant des molécules anti-sens et/ou mutées à dominant négatif, dans lequel la cellule hôte est en particulier une cellule humaine,
pour une utilisation dans le traitement, la réduction et/ou la prévention du diabète, et/ou de l'obésité.

9. Kit comprenant au moins
(a) une molécule d'acide nucléique SF6 ou un isoforme de celui-ci;
(b) une molécule d'acides aminés SF6 ou un isoforme de celui-ci;
(c) un vecteur comprenant l'acide nucléique de (a);
(d) une cellule hôte comprenant l'acide nucléique de (a) ou le vecteur de (c);
(e) un polypeptide codant pour l'acide nucléique de (a), exprimé par le vecteur de (c) ou de la cellule hôte de (d);
(f) un polypeptide de fusion codant pour l'acide nucléique de (a);
(g) un anticorps, un aptamère ou un autre effecteur/modulateur de l'acide nucléique de (a) ou du polypeptide de (b), (e), ou (f) et/ou
(h) un oligonucléotide anti-sens de l'acide nucléique de (a).


### SEQUENCE LISTING

<110> DeveloGen Aktiengesellschaft
<120> Use of secreted protein products for preventing and treating pancreatic diseases and/or obesity and/or metabolic syndrome
<130> 31993P EP-WO-1
<140> EP08016231 <141> 2008-09-15
<150> EP 03 026 614.2 <151> 2003-11-19
<160> 28
<170> PatentIn version 3.3
<210> 1
   <211> 20
   <212> DNA
   <213> Artificial
<220>
   <223> mouse SF1 forward primer
<400> 1
   gttgccaagg tctttgggag 20
<210> 2
   <211> 22
   <212> DNA
   <213> Artificial
<220>
   <223> mouse SF1 reverse primer
<400> 2
   ctcacgacca cgtctcttca gt 22
<210> 3
   <211> 30
   <212> DNA
   <213> Artificial
<220>
   <223> mouse SF1 Taqman probe
<400> 3
   agttcacaca gatctcacca gtctggttgc 30
<210> 4
   <211> 16
   <212> DNA
   <213> Artificial
<220>
   <223> mouse SF2 forward primer
<400> 4
   gccatggtcc ggctca 16
<210> 5
   <211> 19
   <212> DNA
   <213> Artificial
<220>
   <223> mouse SF2 reverse primer
<400> 5
   ctttgggttg caggcttca 19
<210> 6
   <211> 29
   <212> DNA
   <213> Artificial
<220>
   <223> mouse SF2 Taqman probe
<400> 6
   tgatttcctc catgaccctc tgtcattgt 29
<210> 7
   <211> 20
   <212> DNA
   <213> Artificial
<220>
   <223> mouse SF3 forward primer
<400> 7
   ccgggagaga tacgtgaagc 20
<210> 8
   <211> 18
   <212> DNA
   <213> Artificial
<220>
   <223> mouse SF3 reverse primer
<400> 8
   tctccgtctc ctccgtgg 18
<210> 9
   <211> 23
   <212> DNA
   <213> Artificial
<220>
   <223> mouse SF3 Taqman probe
<400> 9
   ccggaagacg gctcagtgtg cat 23
<210> 10
   <211> 17
   <212> DNA
   <213> Artificial
<220>
   <223> mouse SF4 forward primer
<400> 10
   agctgcctcc gtccctg 17
<210> 11
   <211> 23
   <212> DNA
   <213> Artificial
<220>
   <223> mouse SF4 reverse primer
<400> 11
   ttcagcaatc cttccagaga gac 23
<210> 12
   <211> 24
   <212> DNA
   <213> Artificial
<220>
   <223> mouse SF4 Taqman probe
<400> 12
   ttcggagact ctacgacagc cgcc 24
<210> 13
   <211> 20
   <212> DNA
   <213> Artificial
<220>
   <223> mouse SF5 forward primer
<400> 13
   cgggccaaga caatgacatc 20
<210> 14
   <211> 21
   <212> DNA
   <213> Artificial
<220>
   <223> mouse SF5 reverse primer
<400> 14
   gtctcattgc acacaaggcc t 21
<210> 15
   <211> 24
   <212> DNA
   <213> Artificial
<220>
   <223> mouse SF5 Taqman probe
<400> 15
   actgcctctg ccctgaaggc ttca 24
<210> 16
   <211> 18
   <212> DNA
   <213> Artificial
<220>
   <223> mouse SF6 forward primer
<400> 16
   tcgcttcacc gggtcttc 18
<210> 17
   <211> 18
   <212> DNA
   <213> Artificial
<220>
   <223> mouse SF6 reverse primer
<400> 17
   ggccattctt gcactggc 18
<210> 18
   <211> 25
   <212> DNA
   <213> Artificial
<220>
   <223> mouse SF6 Taqman probe
<400> 18
   atcaagcacg gcacggagtg taccc 25
<210> 19
   <211> 20
   <212> DNA
   <213> Artificial
<220>
   <223> mouse SF7 forward primer
<400> 19
   ccatgctgca actggatgaa 20
<210> 20
   <211> 19
   <212> DNA
   <213> Artificial
<220>
   <223> mouse SF7 reverse primer
<400> 20
   caggtgtaca ggagggcca 19
<210> 21
   <211> 22
   <212> DNA
   <213> Artificial
<220>
   <223> mouse SF7 Taqman probe
<400> 21
   aatgtgctgc ctgctgccac ca 22
<210> 22
   <211> 17
   <212> DNA
   <213> Artificial
<220>
   <223> mouse SF8 forward primer
<400> 22
   tggctgggtc agcatgg 17
<210> 23
   <211> 22
   <212> DNA
   <213> Artificial
<220>
   <223> mouse SF8 reverse primer
<400> 23
   tcaaggtgat gaagaactgg ga 22
<210> 24
   <211> 24
   <212> DNA
   <213> Artificial
<220>
   <223> mouse SF8 Taqman probe
<400> 24
   caatgctgga ccagacacca acgg 24
<210> 25
   <211> 819
   <212> PRT
   <213> Mus musculus
<220>
   <221> MISC_FEATURE
   <223> GenBank Accession No. NP_058023
   nel-like 2 homolog
<400> 25
   Met His Ala Met Glu Ser Arg Val Leu Leu Arg Thr Phe Cys Val Ile
   1               5                   10                  15
   Leu Gly Leu Gly Ala Val Trp Gly Leu Gly Val Asp Pro Ser Leu Gln
               20                  25                  30
   Ile Asp Val Leu Thr Glu Leu Glu Leu Gly Glu Ser Thr Asp Gly Val
           35                  40                  45
   Arg Gln Val Pro Gly Leu His Asn Gly Thr Lys Ala Phe Leu Phe Gln
       50                  55                  60
   Glu Ser Pro Arg Ser Ile Lys Ala Ser Thr Ala Thr Ala Glu Arg Phe
   65                  70                  75                  80
   Phe Gln Lys Leu Arg Asn Lys His Glu Phe Thr Ile Leu Val Thr Leu
                   85                  90                  95
   Lys Gln Ile His Leu Asn Ser Gly Val Ile Leu Ser Ile His His Leu
               100                 105                 110
   Asp His Arg Tyr Leu Glu Leu Glu Ser Ser Gly His Arg Asn Glu Ile
           115                 120                 125
   Arg Leu His Tyr Arg Ser Gly Thr His Arg Pro His Thr Glu Val Phe
       130                 135                 140
   Pro Tyr Ile Leu Ala Asp Ala Lys Trp His Lys Leu Ser Leu Ala Phe
   145                 150                 155                 160
   Ser Ala Ser His Leu Ile Leu His Ile Asp Cys Asn Lys Ile Tyr Glu
                   165                 170                 175
   Arg Val Val Glu Met Pro Ser Thr Asp Leu Pro Leu Gly Thr Thr Phe
               180                 185                 190
   Trp Leu Gly Gln Arg Asn Asn Ala His Gly Tyr Phe Lys Gly Ile Met
           195                 200                 205
   Gln Asp Val His Val Leu Val Met Pro Gln Gly Phe Ile Ala Gln Cys
       210                 215                 220
   Pro Asp Leu Asn Arg Thr Cys Pro Thr Cys Asn Asp Phe His Gly Leu
   225                 230                 235                 240
   Val Gln Lys Ile Met Glu Leu Gln Asp Ile Leu Ser Lys Thr Ser Ala
                   245                 250                 255
   Lys Leu Ser Arg Ala Glu Gln Arg Met Asn Arg Leu Asp Gln Cys Tyr
               260                 265                 270
   Cys Glu Arg Thr Cys Thr Val Lys Gly Thr Thr Tyr Arg Glu Ser Glu
           275                 280                 285
   Ser Trp Thr Asp Gly Cys Lys Asn Cys Thr Cys Leu Asn Gly Thr Ile
       290                 295                 300
   Gln Cys Glu Thr Leu Val Cys Pro Ala Pro Asp Cys Pro Pro Lys Ser
   305                 310                 315                 320
   Ala Pro Ala Tyr Val Asp Gly Lys Cys Cys Lys Glu Cys Lys Ser Thr
                   325                 330                 335
   Cys Gln Phe Gln Gly Arg Ser Tyr Phe Glu Gly Glu Arg Asn Thr Val
               340                 345                 350
   Tyr Ser Ser Ser Gly Met Cys Val Leu Tyr Glu Cys Lys Asp Gln Thr
           355                 360                 365
   Met Lys Leu Val Glu Asn Ile Gly Cys Pro Pro Leu Asp Cys Pro Glu
   370 375 380
   Ser His Gln Ile Ala Leu Ser His Ser Cys Cys Lys Val Cys Lys Gly
   385 390 395 400
   Tyr Asp Phe Cys Ser Glu Lys His Thr Cys Met Glu Asn Ser Val Cys
   405 410 415
   Arg Asn Leu Asn Asp Arg Ala Val Cys Ser Cys Arg Asp Gly Phe Arg
   420 425 430
   Ala Leu Arg Glu Asp Asn Ala Tyr Cys Glu Asp Ile Asp Glu Cys Ala
   435 440 445
   Glu Gly Arg His Tyr Cys Arg Glu Asn Thr Met Cys Val Asn Thr Pro
   450 455 460
   Gly Ser Phe Met Cys Ile Cys Lys Thr Gly Tyr Ile Arg Ile Asp Asp
   465 470 475 480
   Tyr Ser Cys Thr Glu His Asp Glu Cys Leu Thr Asn Gln His Asn Cys
   485 490 495
   Asp Glu Asn Ala Leu Cys Phe Asn Thr Val Gly Gly His Asn Cys Val
   500 505 510
   Cys Lys Pro Gly Tyr Thr Gly Asn Gly Thr Thr Cys Lys Ala Phe Cys
   515 520 525
   Lys Asp Gly Cys Arg Asn Gly Gly Ala Cys Ile Ala Ala Asn Val Cys
   530 535 540
   Ala Cys Pro Gln Gly Phe Thr Gly Pro Ser Cys Glu Thr Asp Ile Asp
   545 550 555 560
   Glu Cys Ser Glu Gly Phe Val Gln Cys Asp Ser Arg Ala Asn Cys Ile
   565 570 575
   Asn Leu Pro Gly Trp Tyr His Cys Glu Cys Arg Asp Gly Tyr His Asp
   580 585 590
   Asn Gly Met Phe Ala Pro Gly Gly Glu Ser Cys Glu Asp Ile Asp Glu
   595 600 605
   Cys Gly Thr Gly Arg His Ser Cys Thr Asn Asp Thr Ile Cys Phe Asn
   610 615 620
   Leu Asp Gly Gly Tyr Asp Cys Arg Cys Pro His Gly Lys Asn Cys Thr
   625 630 635 640
   Gly Asp Cys Val His Glu Gly Lys Val Lys His Thr Gly Gln Ile Trp
   645 650 655
   Val Leu Glu Asn Asp Arg Cys Ser Val Cys Ser Cys Gln Thr Gly Phe
   660 665 670
   Val Met Cys Arg Arg Met Val Cys Asp Cys Glu Asn Pro Thr Val Asp
   675 680 685
   Leu Ser Cys Cys Pro Glu Cys Asp Pro Arg Leu Ser Ser Gln Cys Leu
   690 695 700
   His Gln Asn Gly Glu Thr Val Tyr Asn Ser Gly Asp Thr Trp Val Gln
   705 710 715 720
   Asp Cys Arg Gln Cys Arg Cys Leu Gln Gly Glu Val Asp Cys Trp Pro
   725 730 735
   Leu Ala Cys Pro Glu Val Glu Cys Glu Phe Ser Val Leu Pro Glu Asn
   740 745 750
   Glu Cys Cys Pro Arg Cys Val Thr Asp Pro Cys Gln Ala Asp Thr Ile
   755 760 765
   Arg Asn Asp Ile Thr Lys Thr Cys Leu Asp Glu Met Asn Val Val Arg
   770 775 780
   Phe Thr Gly Ser Ser Trp Ile Lys His Gly Thr Glu Cys Thr Leu Cys
   785 790 795 800
   Gln Cys Lys Asn Gly His Leu Cys Cys Ser Val Asp Pro Gln Cys Leu
   805 810 815
   Gln Glu Leu
<210> 26
   <211> 816
   <212> PRT
   <213> Homo sapiens
<220>
   <221> MISC_FEATURE
   <223> GenBan Accession No. NP_006150
   nel-like 2
<400> 26
   Met Glu Ser Arg Val Leu Leu Arg Thr Phe Cys Leu Ile Phe Gly Leu
   1 5 10 15
   Gly Ala Val Trp Gly Leu Gly Val Asp Pro Ser Leu Gln Ile Asp Val
   20 25 30
   Leu Thr Glu Leu Glu Leu Gly Glu Ser Thr Thr Gly Val Arg Gln Val
               35                  40                  45
   Pro Gly Leu His Asn Gly Thr Lys Ala Phe Leu Phe Gln Asp Thr Pro
       50                  55                  60
   Arg Ser Ile Lys Ala Ser Thr Ala Thr Ala Glu Gln Phe Phe Gln Lys
   65                  70                  75                  80
   Leu Arg Asn Lys His Glu Phe Thr Ile Leu Val Thr Leu Lys Gln Thr
                   85                  90                  95
   His Leu Asn Ser Gly Val Ile Leu Ser Ile His His Leu Asp His Arg
               100                 105                 110
   Tyr Leu Glu Leu Glu Ser Ser Gly His Arg Asn Glu Val Arg Leu His
           115                 120                 125
   Tyr Arg Ser Gly Ser His Arg Pro His Thr Glu Val Phe Pro Tyr Ile
       130                 135                 140
   Leu Ala Asp Asp Lys Trp His Lys Leu Ser Leu Ala Ile Ser Ala Ser
   145                 150                 155                 160
   His Leu Ile Leu His Ile Asp Cys Asn Lys Ile Tyr Glu Arg Val Val
                   165                 170                 175
   Glu Lys Pro Ser Thr Asp Leu Pro Leu Gly Thr Thr Phe Trp Leu Gly
               180                 185                 190
   Gln Arg Asn Asn Ala His Gly Tyr Phe Lys Gly Ile Met Gln Asp Val
           195                 200                 205
   Gln Leu Leu Val Met Pro Gln Gly Phe Ile Ala Gln Cys Pro Asp Leu
       210                 215                 220
   Asn Arg Thr Cys Pro Thr Cys Asn Asp Phe His Gly Leu Val Gln Lys
   225                 230                 235                 240
   Ile Met Glu Leu Gln Asp Ile Leu Ala Lys Thr Ser Ala Lys Leu Ser
                   245                 250                 255
   Arg Ala Glu Gln Arg Met Asn Arg Leu Asp Gln Cys Tyr Cys Glu Arg
               260                 265                 270
   Thr Cys Thr Met Lys Gly Thr Thr Tyr Arg Glu Phe Glu Ser Trp Ile
           275                 280                 285
   Asp Gly Cys Lys Asn Cys Thr Cys Leu Asn Gly Thr Ile Gln Cys Glu
       290                 295                 300
   Thr Leu Ile Cys Pro Asn Pro Asp Cys Pro Leu Lys Ser Ala Leu Ala
   305                 310                 315                 320
   Tyr Val Asp Gly Lys Cys Cys Lys Glu Cys Lys Ser Ile Cys Gln Phe
                   325                 330                 335
   Gln Gly Arg Thr Tyr Phe Glu Gly Glu Arg Asn Thr Val Tyr Ser Ser
               340                 345                 350
   Ser Gly Val Cys Val Leu Tyr Glu Cys Lys Asp Gln Thr Met Lys Leu
           355                 360                 365
   Val Glu Ser Ser Gly Cys Pro Ala Leu Asp Cys Pro Glu Ser His Gln
       370                 375                 380
   Ile Thr Leu Ser His Ser Cys Cys Lys Val Cys Lys Gly Tyr Asp Phe
   385                 390                 395                 400
   Cys Ser Glu Arg His Asn Cys Met Glu Asn Ser Ile Cys Arg Asn Leu
                   405                 410                 415
   Asn Asp Arg Ala Val Cys Ser Cys Arg Asp Gly Phe Arg Ala Leu Arg
               420                 425                 430
   Glu Asp Asn Ala Tyr Cys Glu Asp Ile Asp Glu Cys Ala Glu Gly Arg
           435                 440                 445
   His Tyr Cys Arg Glu Asn Thr Met Cys Val Asn Thr Pro Gly Ser Phe
       450                 455                 460
   Met Cys Ile Cys Lys Thr Gly Tyr Ile Arg Ile Asp Asp Tyr Ser Cys
   465                 470                 475                 480
   Thr Glu His Asp Glu Cys Ile Thr Asn Gln His Asn Cys Asp Glu Asn
                   485                 490                 495
   Ala Leu Cys Phe Asn Thr Val Gly Gly His Asn Cys Val Cys Lys Pro
               500                 505                 510
   Gly Tyr Thr Gly Asn Gly Thr Thr Cys Lys Ala Phe Cys Lys Asp Gly
           515                 520                 525
   Cys Arg Asn Gly Gly Ala Cys Ile Ala Ala Asn Val Cys Ala Cys Pro
       530                 535                 540
   Gln Gly Phe Thr Gly Pro Ser Cys Glu Thr Asp Ile Asp Glu Cys Ser
   545                 550                 555                 560
   Asp Gly Phe Val Gln Cys Asp Ser Arg Ala Asn Cys Ile Asn Leu Pro
                   565                 570                 575
   Gly Trp Tyr His Cys Glu Cys Arg Asp Gly Tyr His Asp Asn Gly Met
               580                 585                 590
   Phe Ser Pro Ser Gly Glu Ser Cys Glu Asp Ile Asp Glu Cys Gly Thr
           595                 600                 605
   Gly Arg His Ser Cys Ala Asn Asp Thr Ile Cys Phe Asn Leu Asp Gly
       610                 615                 620
   Gly Tyr Asp Cys Arg Cys Pro His Gly Lys Asn Cys Thr Gly Asp Cys
   625                 630                 635                 640
   Ile His Asp Gly Lys Val Lys His Asn Gly Gln Ile Trp Val Leu Glu
                   645                 650                 655
   Asn Asp Arg Cys Ser Val Cys Ser Cys Gln Asn Gly Phe Val Met Cys
               660                 665                 670
   Arg Arg Met Val Cys Asp Cys Glu Asn Pro Thr Val Asp Leu Phe Cys
           675                 680                 685
   Cys Pro Glu Cys Asp Pro Arg Leu Ser Ser Gln Cys Leu His Gln Asn
       690                 695                 700
   Gly Glu Thr Leu Tyr Asn Ser Gly Asp Thr Trp Val Gln Asn Cys Gln
   705                 710                 715                 720
   Gln Cys Arg Cys Leu Gln Gly Glu Val Asp Cys Trp Pro Leu Pro Cys
                   725                 730                 735
   Pro Asp Val Glu Cys Glu Phe Ser Ile Leu Pro Glu Asn Glu Cys Cys
               740                 745                 750
   Pro Arg Cys Val Thr Asp Pro Cys Gln Ala Asp Thr Ile Arg Asn Asp
           755                 760                 765
   Ile Thr Lys Thr Cys Leu Asp Glu Met Asn Val Val Arg Phe Thr Gly
       770                 775                 780
   Ser Ser Trp Ile Lys His Gly Thr Glu Cys Thr Leu Cys Gln Cys Lys
   785                 790                 795                 800
   Asn Gly His Ile Cys Cys Ser Val Asp Pro Gln Cys Leu Gln Glu Leu
                   805                 810                 815
<210> 27
   <211> 3385
   <212> DNA
   <213> Mus musculus
<220>
   <221> misc_feature
   <223> GenBank Accession No. NM_016743
   NEL-like 2 (Ne112)
<400> 27
   cccgcgcctg tccagcttca cctaggagtc ttgcgtgctt tggctgggtt ccttttcccg 60
   cccgggaggg ggtgccctgc gccggggctg ccgtgcagcg tgagtgtcta agtgagggct 120
   tccctctttt gcccgaggcg gccgggtgcc tttctttgca acctcgcctc ctgcggctgg 180
   gtggtccttt ctctcgccgg gctttggaga cacgcgcccg atttcgaggg gagggagacg 240
   atggactgag acgatgcacg ccatggaatc ccgggtgtta ctgagaacgt tctgcgtgat 300
   cctcgggctt ggagcggttt gggggcttgg tgtggacccc tccctacaga ttgacgtctt 360
   aacagagtta gaacttgggg agtctacaga tggagtgcgc caagtcccgg gactgcataa 420
   tgggacgaaa gccttcctct tccaagagtc ccccagaagc ataaaggcat ccactgctac 480
   agctgagcgg tttttccaga agctgagaaa taaacacgag ttcacaatct tggtgaccct 540
   gaaacagatc cacttaaatt cgggagttat cctctccatc caccacttgg atcacaggta 600
   cctggaactg gaaagcagtg gccatcggaa tgagatcaga ctccactacc gctctggcac 660
   tcaccgtccc cacacggaag tgtttcctta tattttggct gatgccaagt ggcacaagct 720
   ctccttagcc ttcagtgcct ctcacttaat tttacacatc gactgcaata agatctatga 780
   acgagtggtg gaaatgcctt caacagactt gcctctgggc accacatttt ggttgggaca 840
   gagaaataat gcacatggct attttaaggg aataatgcag gatgtgcacg ttcttgtcat 900
   gcctcagggc ttcattgctc agtgcccgga ccttaatcga acctgtccaa catgcaacga 960
   cttccatggg cttgtgcaga aaatcatgga gctgcaggac attttatcaa agacgtcagc 1020
   caagttgtcc agagctgaac aaagaatgaa caggctggat cagtgctact gtgagcggac 1080
   atgcactgtg aagggaacca cctaccgaga gtctgagtcc tggacagacg gctgtaagaa 1140
   ctgcacatgc ttgaacggga ccatccagtg cgagactctg gtctgccctg ctcctgactg 1200
   ccctcctaaa tcggcccctg cgtatgtgga tggcaagtgc tgtaaggagt gcaaatcaac 1260
   ctgccagttc cagggacgga gctactttga gggagaaagg aacacggtat actcatcttc 1320
   tggaatgtgt gtcttatatg aatgcaagga tcagaccatg aagcttgttg agaacattgg 1380
   ctgcccaccc ttagattgtc ccgagtctca tcagattgcc ttgtctcaca gctgctgcaa 1440
   ggtttgtaaa ggttatgact tctgttctga gaagcatacc tgcatggaga actcggtctg 1500
   caggaacctg aacgacaggg ctgtgtgcag ctgcagggat ggttttcggg ctctccgaga 1560
   ggacaacgcc tactgtgaag acattgacga gtgtgcagaa gggcgccatt actgccgtga 1620
   gaacaccatg tgtgtgaata cacctggttc tttcatgtgt atctgcaaaa ctgggtacat 1680
   caggatcgac gattactcat gtacagaaca tgatgagtgt ctcacaaacc agcacaattg 1740
   tgatgaaaac gctttgtgct ttaacactgt tggaggacac aactgtgtct gcaagcctgg 1800
   ctacaccggg aatggaacca cgtgcaaagc tttctgcaaa gatggctgta gaaacggagg 1860
   agcgtgcatt gctgccaatg tgtgtgcctg cccacaaggc ttcacgggac ccagctgtga 1920
   gacagacatt gacgagtgct ctgagggctt tgttcagtgt gacagccgtg ccaactgcat 1980
   caacctgcct gggtggtatc actgtgagtg cagagacggc taccatgaca atgggatgtt 2040
   tgcgccaggc ggagaatcct gtgaagatat tgacgaatgc gggactggga ggcacagctg   2100
   caccaacgac accatttgct tcaacttgga cgggggatac gattgccggt gtccccatgg   2160
   gaagaactgc actggggact gcgtgcacga ggggaaagtg aagcacaccg gccagatctg   2220
   ggtgctggaa aacgacaggt gctccgtgtg ttcctgccag actgggtttg tcatgtgtcg   2280
   acggatggtc tgcgactgcg aaaaccccac agttgacctt tcctgctgcc ctgagtgtga   2340
   cccaaggctg agcagtcagt gcctgcatca aaacggggaa accgtgtaca acagcggcga   2400
   cacctgggtc caggattgcc gtcagtgccg ctgcttgcaa ggagaagttg actgttggcc   2460
   cctggcttgc ccagaggtag aatgtgaatt tagcgtcctt cctgagaacg agtgctgccc   2520
   acgctgtgtc accgatcctt gtcaggccga caccatccgc aatgacatca ccaaaacctg   2580
   cctggacgag atgaacgtgg ttcgcttcac cgggtcttcc tggatcaagc acggcacgga   2640
   gtgtaccctc tgccagtgca agaatggcca tttgtgctgc tcagtggatc cacagtgcct   2700
   tcaggagctg tgaagttaac tgcctcatgg gagatatctg ttcaaagaat gttttctcat   2760
   ttcaaaagac caaataaata aataaataaa taaaaagtga ggtgtggcca gccagatgca   2820
   actttgttag cagctggata gactgatgtc gattatggat tcgtggggcc tgaggaacat   2880
   ctctgaggaa gcgagatgac cattctgctt ttactattcc tgggatcacc ttgcagagga   2940
   atgggtgtgg agcccaggcc atgccatcct cgcccctgga taagaagcct gagccccatc   3000
   agctctggga gagcctctcg ctctgtccgc agccaggcgc aggacacgtc cttctatctc   3060
   agactcttcc tgagtcagcg aggctcctca ctgaggctgt cgaacgtgag gtgacagcgg   3120
   tgagcgagtt atattttcag aatccaagaa gctgatgcgt ctgtacagtg cactccgcaa   3180
   cccgaaacaa gctattgtca tgataaataa cgcacaggca tggttacgta accttttcta   3240
   acacgaaaag tcacccctcc cccccccccc cagtttcctt gtttactgcc cttaatattg   3300
   tttggttgaa tttgttcagt agacgcttgt tcttgtgcaa aataaaataa ctatttctct   3360
   taccttaaaa aaaaaaaaaa aaaaa                                          3385
<210> 28
   <211> 3198
   <212> DNA
   <213> Homo sapiens
<220>
   <221> misc_feature
   <223> GenBank Accession No. NM_006159
   NEL-like 2 (NELL2)
<400> 28
   ttgggaggag cagtctctcc gctcgtctcc cggagctttc tccattgtct ctgcctttac     60
   aacagaggga gacgatggac tgagctgatc cgcaccatgg agtctcgggt cttactgaga    120
   acattctgtt tgatcttcgg tctcggagca gtttgggggc ttggtgtgga cccttcccta    180
   cagattgacg tcttaacaga gttagaactt ggggagtcca cgaccggagt gcgtcaggtc    240
   ccggggctgc ataatgggac gaaagccttt ctctttcaag atactcccag aagcataaaa    300
   gcatccactg ctacagctga acagtttttt cagaagctga gaaataaaca tgaatttact    360
   attttggtga ccctaaaaca gacccactta aattcaggag ttattctctc aattcaccac    420
   ttggatcaca ggtacctgga actggaaagt agtggccatc ggaatgaagt cagactgcat    480
   taccgctcag gcagtcaccg ccctcacaca gaagtgtttc cttacatttt ggctgatgac    540
   aagtggcaca agctctcctt agccatcagt gcttcccatt tgattttaca cattgactgc    600
   aataaaattt atgaaagggt agtagaaaag ccctccacag acttgcctct aggcacaaca    660
   ttttggctag gacagagaaa taatgcgcat ggatatttta agggtataat gcaagatgtc    720
   caattacttg tcatgcccca gggatttatt gctcagtgcc cagatcttaa tcgcacctgt    780
   ccaacttgca atgacttcca tggacttgtg cagaaaatca tggagctaca ggatatttta    840
   gccaaaacat cagccaagct gtctcgagct gaacagcgaa tgaatagatt ggatcagtgc    900
   tattgtgaaa ggacttgcac catgaaggga accacctacc gagaatttga gtcctggata    960
   gacggctgta agaactgcac atgcctgaat ggaaccatcc agtgtgaaac tctaatctgc   1020
   ccaaatcctg actgcccact taagtcggct cttgcgtatg tggatggcaa atgctgtaag   1080
   gaatgcaaat cgatatgcca atttcaagga cgaacctact ttgaaggaga aagaaataca   1140
   gtctattcct cttctggagt atgtgttctc tatgagtgca aggaccagac catgaaactt   1200
   gttgagagtt caggctgtcc agctttggat tgtccagagt ctcatcagat aaccttgtct   1260
   cacagctgtt gcaaagtttg taaaggttat gacttttgtt ctgaaaggca taactgcatg   1320
   gagaattcca tctgcagaaa tctgaatgac agggctgttt gtagctgtcg agatggtttt   1380
   agggctcttc gagaggataa tgcctactgt gaagacatcg atgagtgtgc tgaagggcgc   1440
   cattactgtc gtgaaaatac aatgtgtgtc aacaccccgg gttcttttat gtgcatctgc   1500
   aaaactggat acatcagaat tgatgattat tcatgtacag aacatgatga gtgtatcaca   1560
   aatcagcaca actgtgatga aaatgcttta tgcttcaaca ctgttggagg acacaactgt   1620
   gtttgcaagc cgggctatac agggaatgga acgacatgca aagcattttg caaagatggc   1680
   tgtaggaatg gaggagcctg tattgccgct aatgtgtgtg cctgcccaca aggcttcact   1740
   ggacccagct gtgaaacgga cattgatgaa tgctctgatg gttttgttca atgtgacagt   1800
   cgtgctaatt gcattaacct gcctggatgg taccactgtg agtgcagaga tggctaccat   1860
   gacaatggga tgttttcacc aagtggagaa tcgtgtgaag atattgatga gtgtgggacc   1920
   gggaggcaca gctgtgccaa tgataccatt tgcttcaatt tggatggcgg atatgattgt   1980
   cgatgtcctc atggaaagaa ttgcacaggg gactgcatcc atgatggaaa agttaagcac   2040
   aatggtcaga tttgggtgtt ggaaaatgac aggtgctctg tgtgctcatg tcagaatgga   2100
   ttcgttatgt gtcgacggat ggtctgtgac tgtgagaatc ccacagttga tcttttttgc   2160
   tgccctgaat gtgacccaag gcttagtagt cagtgcctcc atcaaaatgg ggaaactttg   2220
   tataacagtg gtgacacctg ggtccagaat tgtcaacagt gccgctgctt gcaaggggaa   2280
   gttgattgtt ggcccctgcc ttgcccagat gtggagtgtg aattcagcat tctcccagag   2340
   aatgagtgct gcccgcgctg tgtcacagac ccttgccagg ctgacaccat ccgcaatgac   2400
   atcaccaaga cttgcctgga cgaaatgaat gtggttcgct tcaccgggtc ctcttggatc   2460
   aaacatggca ctgagtgtac tctctgccag tgcaagaatg gccacatctg ttgctcagtg   2520
   gatccacagt gccttcagga actgtgaagt taactgtctc atgggagatt tctgttaaaa   2580
   gaatgttctt tcattaaaag accaaaaaga agttaaaact taaattgggt gatttgtggg   2640
   cagctaaatg cagctttgtt aatagctgag tgaactttca attatgaaat ttgtggagct   2700
   tgacaaaatc acaaaaggaa aattactggg gcaaaattag acctcaagtc tgcctctact   2760
   gtgtctcaca tcaccatgta gaagaatggg cgtacagtat ataccgtgac atcctgaacc   2820
   ctggatagaa agcctgagcc cattggatct gtgaaagcct ctagcttcac tggtgcagaa   2880
   aattttcctc tagatcagaa tcttcagaat cagttaggtt cctcactgca agaaataaaa   2940
   tgtcaggcag tgaatgaatt atattttcag aagtaaagca aagaagctat aacatgttat   3000
   gtacagtaca ctctgaaaag aaatctgaaa caagttattg taatgataaa aataatgcac   3060
   aggcatggtt acttaatatt ttctaacagg aaaagtcatc cctatttcct tgttttactg   3120
   cacttaatat tatttggttg aatttgttca gtataagctc gttcttgtgc aaaattaaat   3180
   aaatatttct cttacctt                                                 3198